# EUROPÄISCHE PATENTSCHRIFT

(11) **EP 3 507 357 B1**
(45) Veröffentlichungstag und Bekanntmachung des Hinweises auf die Patenterteilung: **11.10.2023**
(21) Anmeldenummer: 17765365.6
(22) Anmeldetag: 30.08.2017
(51) Int. Cl.: C12M 1/00, C12M 1/12

(54) **PHOTOBIOREAKTOR MIT BEWEGLICHER INSTANDHALTUNGSVORRICHTUNG**
PHOTOBIOREACTOR AND METHOD FOR CULTIVATING MICROALGAE
PHOTOBIOREACTEUR ET PROCEDE DE CULTURE DE MICRO-ALGUES

(30) Priorität: 30.08.2016 EP 16186440; 08.02.2017 EP 17155258
(43) Veröffentlichungstag der Anmeldung: 10.07.2019
(73) Patentinhaber: Beco Invest B.V., 3992 LX Houten (NL)
(72) Erfinder: EMMINGER, Franz, 2410 Hainburg (AT); FLUCH, Silvia, 7121 Weiden/See (AT)
(74) Vertreter: Redl, Gerda
(86) Internationale Anmeldenummer: PCT/EP2017/071702
(87) Internationale Veröffentlichungsnummer: WO 2018/041859

(56) Entgegenhaltungen:
- EP-A1- 2 947 139
- EP-A2- 1 632 562
- WO-A1-2015/179888
- CN-U- 202 968 549

## Beschreibung

Das Gebiet der vorliegenden Erfindung ist das der Rohr-Photobioreaktoren zur Kultivierung phototropher Mikroorganismen wie z.B. Mikroalgen, wobei die Rohr-Photobioreaktoren mit einer Rohr-Instandhaltungsvorrichtung ausgestattet sind.

Phototrophe Mikroorganismen wie Mikroalgen werden vor allem ihrer wertvollen Inhaltsstoffe wegen kultiviert, die z.B. in der Herstellung von medizinischen Präparaten, Nahrungs- und Futtermitteln, Nahrungsergänzungsmitteln, und Kosmetika benötigt werden. Zu diesen Inhaltsstoffen gehören unter anderem ungesättigte Fettsäuren (z.B. Omega-3 und Omega-6-Fettsäuren), Antioxidantien wie Astaxanthin und Lutein, und Chlorophyll. Die Zusammensetzung der Inhaltsstoffe ist von der kultivierten Art von phototrophen Mikroorganismen abhängig. Vorwiegend wird eine Reinkultur von phototrophen Mikroorganismen herangezogen, um eine gewisse Qualität bzw. Mindestkonzentration der gewünschte Inhaltsstoffe gewährleisten zu können.

Üblicherweise werden die Photosynthese betreibenden Mikroorganismen unter Einwirkung von Licht (z.B. Sonnenlicht) im nährstoffhaltigen Kulturmedium in einem Photobioreaktor kultiviert, und anschließend aufkonzentriert und verarbeitet. Aus der erhaltenen Biomasse können die Inhaltsstoffe zur Weiterverarbeitung gewonnen werden bzw. kann die Biomasse selbst z.B. als Futtermittel oder Dünger verwendet werden. So offenbaren beispielsweise die WO 2015/179888 A1 einen Photobioreaktor bzw. die WO 2016/168871 A2 oder die CN 202 968 549 U eine Erntevorrichtung für Algen.

Im Allgemeinen sind die Rohre eines Photobioreaktors insbesondere im Dauerbetrieb anfällig dafür, mit der Zeit zu verschmutzen. Dabei lagern sich oft Rückstände aus der Kultivierung (beispielsweise aus Überresten abgestorbener Mikroorganismen) an der Innenseite der Rohre an. Dies kann zum einen die Lichtausbeute mindern (da diese Rückstände typischerweise Licht absorbieren) und zum anderen eine ständige Gefahr in Bezug auf Kontamination des Kulturmediums mit unerwünschten Mikroorganismen darstellen, die in den Rückständen einen geeigneten Nährboden finden. Durch verminderte Lichtausbeute bzw. Kontamination kann sich wiederum die Ausbeute aus der Kultivierung deutlich verringern. Des weiteren kann es insbesondere für sensible Bereiche wie die Herstellung von medizinischen Präparaten oder Nahrungsergänzungsmitteln von hoher Bedeutung für die Produktsicherheit sein, Kontaminationen der Kultur mit unerwünschten Mikroorganismen möglichst auszuschließen.

Im Stand der Technik werden verschiedene Lösungen für die obig erläuterte Problematik vorgeschlagen.

Die WO 94/09112 A1 offenbart ein Gerät zur Reinigung von Photobioreaktor-Rohren und einen Photobioreaktor, der selbiges Gerät umfasst. Im Wesentlichen bewegt dieses Gerät mobile Reinigungselemente (z.B. Kugeln, die an der Innenoberfläche des Rohres reiben) mit einem magnetischen Kern, die wiederum durch einen "Kolbeneffekt" das Kulturmedium vor sich hertreiben. Dadurch kann gemäß dem Dokument auf Pumpen oder Erzeugung einer Strömung durch "Gaslift" verzichtet werden.

Die EP 2 947 139 A1 betrifft einen Reaktor zur photochemischen Züchtung einer flüssigen Kultur von Klein- und Kleinstlebewesen, insbesondere Algen, ein Reinigungsverfahren für diesen sowie dessen Verwendung. Dabei werden im Wesentlichen zur Reinigung durch die Pumpe des Reaktors Schlitten mit je einem Reinigungsteil entlang der Fließrichtung in den Rohren des Reaktors bewegt.

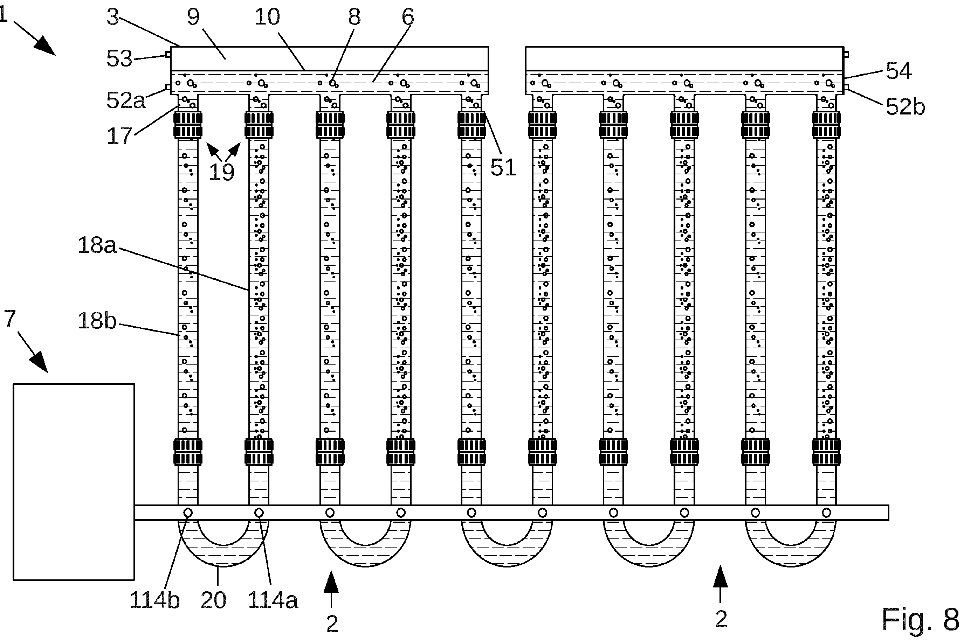

Die WO 2010/025345 A2 bezieht sich auf einen "Semi-Closed Loop"-Photobioreaktor zur Herstellung von Dieseltreibstoff aus Algen unter Verwendung von Abwasser. Darin wird ein Reinigungsgerät, das z.B. als Reinigungsbürstengerät ausgebildet sein kann, offenbart, welches einen magnetischen Kern aufweist und von einem außerhalb des Photobioreaktor-Rohres befindlichen Magneten bewegt werden kann (vgl. Fig 8 des Dokuments). Das Dokument lehrt in Absatz [0041], dass der Photobioreaktor nach der Ernte der Algen durchgespült und gereinigt wird (also zwischen den Betriebszyklen, in denen Algen kultiviert werden).

Die WO 2014/133793 A1 betrifft einen modularen Rohr-Bioreaktor.

Darin offenbart ist ein Reinigungsmodul, das einen Molch, Schrubber oder Reinigungsperlen umfassen kann, der oder die mit dem strömenden Kulturmedium mit zirkulieren können.

Die EP 1 632 562 A2 bezieht sich im Wesentlichen auf einen Rohr-Photobioreaktor mit mäanderförmigem Strömungsverlauf, zwischen dessen Rohren Verbindungsstücke vorgesehen sind, in deren inneren Bereich Reinigungsschieber angeordnet sind. Nach einer Ausgestaltung kann der Reinigungsschieber durch eine Inspektionsöffnung mit einer steckbaren Schubstange ins Rohr geschoben werden.

Trotz dieser Lösungsverschläge besteht der Bedarf für Weiterentwicklungen, die beispielsweise die Skalierbarkeit und Lebensdauer von Photobioreaktoren steigern.

Es ist Aufgabe der vorliegenden Erfindung, das Zusammenspiel zwischen Photobioreaktor und Instandhaltungsvorrichtung (die z.B. eine Reinigungsfunktion aufweisen kann) bzw. den Photobioreaktor oder die Instandhaltungsvorrichtung selbst weiter zu verbessern, so dass Lebensdauer und/oder Ausbeute des Photobioreaktors erhöht bzw. der Wartungsaufwand verringert wird.

Die vorliegende Erfindung ist in den Ansprüchen definiert.

Daher stellt die vorliegende Erfindung einen Photobioreaktor zur Kultivierung phototropher Mikroorganismen, mit einem Reaktorelement, das ein Rohr aufweist, und ferner mit einer Instandhaltungsvorrichtung und einem (bevorzugt externen) Antriebssystem, das die Instandhaltungsvorrichtung im Rohr bewegen kann, zur Verfügung. Der Photobioreaktor ist derart ausgelegt, dass das Rohr im Betriebszustand des Photobioreaktors von flüssigem Kulturmedium mit den Mikroorganismen zumindest teilweise durchströmt wird. Darüber hinaus ist der Photobioreaktor erfindungsgemäß derart ausgelegt, dass die Instandhaltungsvorrichtung im Betriebszustand des Photobioreaktors im Rohr einsetzbar ist und vom Antriebssystem zumindest gegen die Strömung des Kulturmediums im Rohr bewegt werden kann.

Dementsprechend stellt die Erfindung auch ein Verfahren zur Kultivierung phototropher Mikroorganismen in einem Photobioreaktor zur Verfügung. Dieser Photobioreaktor ist in den Ansprüchen beschrieben.

Anders als bei im Stand der Technik bekannten Vorschlägen zur Instandhaltung eines Photobioreaktors, nach denen z.B. Reinigungsperlen mit der Strömung im Photobioreaktor (d.h. "passiv") mitzirkulieren sollen, um die Reaktorwände sauber zu halten, sieht die vorliegende Erfindung wie oben beschrieben im Betriebszustand des Photobioreaktors eine Beweglichkeit der Instandhaltungsvorrichtung zumindest gegen die Strömung des Kulturmediums, üblicherweise entlang der Längsachse des Rohres, vor (zweckmäßigerweise kann die Instandhaltungsvorrichtung vom Antriebssystem auch in Richtung der Strömung des Kulturmediums, üblicherweise entlang der Längsachse des Rohres, bewegt werden). So können Positionen im Rohr (z.B. mit dringend zu beseitigenden Verunreinigungen) unabhängig von der Strömung erreicht werden (d.h. z.B. ohne dass eine Zirkulation durch den gesamten Photobioreaktor erforderlich ist, so dass Positionen schneller erreicht werden können). Damit werden letztlich verunreinigungsbedingte Außerbetriebnahmen vermieden und die Ausbeute des Photobioreaktors erhöht.

Auch die Instandhaltungsvorrichtung selbst ist üblicherweise von Verunreinigungen betroffen (so können sich z.B. Rückstände von abgestorbenen Mikroorganismen auf Oberflächen der Instandhaltungsvorrichtung ansetzen). Herkömmlicherweise muss deshalb eine Instandhaltungsvorrichtung aus dem Photobioreaktor entnommen werden, um sie zu reinigen. Im Zuge der vorliegenden Erfindung hat es sich jedoch als besonders vorteilhaft herausgestellt, wenn der Photobioreaktor ferner eine Einrichtung zur Begasung des Kulturmediums aufweist, wobei der Photobioreaktor derart ausgelegt ist, dass im Betriebszustand des Photobioreaktors die Instandhaltungsvorrichtung im Rohr mit im (begasten) Kulturmedium befindlichen Gasblasen in Berührung gebracht wird. Die durch ihren Auftrieb allmählich im Kulturmedium aufsteigenden Gasblasen verlangsamen bzw. verhindern das Ansetzen von Rückständen auf Oberflächen der Instandhaltsvorrichtung, wodurch die Häufigkeit der Entnahmen der Instandhaltungsvorrichtung aus dem Photobioreaktor zu deren Reinigung verringert werden kann.

Der erfindungsgemäße Photobioreaktor ist in einer bevorzugten Ausführungsform derart ausgelegt, dass im Betriebszustand im Rohr sowohl Kulturmedium als auch, oberhalb des Kulturmediums, ein Gasraum zur Aufnahme von aus dem Kulturmedium aufsteigenden Gasblasen vorhanden sind, wobei im Rohr eine Grenzfläche zwischen dem Kulturmedium und dem Gasraum angeordnet ist. Es hat sich allerdings während der Entwicklung dieser Ausführung herausgestellt, dass gerade die mit der Grenzfläche zwischen Kulturmedium und Gasraum in Berührung befindlichen Innenoberflächen des Rohres (bzw. der "Grenzflächenraum" in der näheren Umgebung der Grenzfläche) anfällig für Verschmutzungen sind, wobei sich mit der Dauer des Betriebes üblicherweise immer mehr Verschmutzungen anlagern. Im Bereich der Grenzfläche (die oft über die Betriebsdauer geringfügig in der Höhe variiert) kann es immer wieder zu Schaumbildung und Ablagerung von Rückständen der phototrophen Mikroorganismen kommen. Zusätzlich kommt es mitunter vor, dass die Innenoberfläche des Photobioreaktors mit Kulturmedium bespritzt wird und diese Spritzer über die Zeit ebenfalls antrocknen und dadurch Verschmutzungen bilden. Daher ist die Instandhaltungsvorrichtung in dieser Ausführung zumindest zur Reinigung von mit dem Gasraum in Berührung befindlicher innerer Oberfläche des Rohres (also für die besonders anfälligen Bereiche des Rohres) eingerichtet. Zweckmäßigerweise ist der Gasraum im als Verteilrohr ausgebildeten Rohr (welcher Gasraum sich oberhalb des Kulturmediums im Verteilrohr befindet) bevorzugt ein Gasraum, der sich entlang der Längsachse des Verteilrohres über zumindest 10%, bevorzugt zumindest 20% oder gar zumindest 30%, mehr bevorzugt zumindest 40% oder gar zumindest 50%, noch mehr bevorzugt zumindest 60% oder gar zumindest 70%, insbesondere zumindest 80% oder gar zumindest 90% der Länge des Verteilrohres erstreckt. Besonders zweckmäßig ist es, wenn sich der genannte Gasraum über die gesamte Länge des Verteilrohres erstreckt. Es versteht sich, dass unter "Gas" auch ein Gasgemisch, insbesondere ein Gemisch eines Gases (z.B. Kohlendioxid) mit Luft, verstanden werden kann.

Vorteilhafterweise weist die Instandhaltungsvorrichtung des erfindungsgemäßen Photobioreaktors eine Sprühdüse zum Besprühen der inneren Oberfläche des Rohres mit einer Reinigungsflüssigkeit auf. Dies hat sich im Dauerbetrieb als schonender und hygienischer im Vergleich zu einer Ausstattung mit Bürsten erwiesen. Besonders bevorzugt ist dabei, wenn die Instandhaltungsvorrichtung zumindest zum Besprühen von mit dem genannten Gasraum in Berührung befindlicher innerer Oberfläche des Rohres durch die Sprühdüse eingerichtet ist. Auf diese Weise können Ablagerungen (die sich wie oben geschildert im Laufe der Zeit ergeben können) besser beseitigt werden.

In einer weiteren bevorzugten Ausführungsform weist die Instandhaltungsvorrichtung zumindest ein Wischblatt aus einem elastischen Material wie beispielsweise einem Kautschuk zum Wischen der inneren Oberfläche des Rohres auf. Auch dies hat sich im Dauerbetrieb als schonender und hygienischer im Vergleich zu einer Ausstattung mit Bürsten erwiesen. Besonders bevorzugt ist dabei, wenn die Instandhaltungsvorrichtung zumindest zum Wischen von mit dem genannten Gasraum in Berührung befindlicher innerer Oberfläche des Rohres mit dem Wischblatt eingerichtet ist. Auf diese Weise können vorhandene Verschmutzungen (die sich wie oben geschildert im Laufe der Zeit ergeben können) besser beseitigt werden. Alternativ, oder zusätzlich, weist die Instandhaltungsvorrichtung bevorzugt eine, gegebenenfalls weitere, Sprühdüse auf, die zum Besprühen des Wischblatts mit einer Reinigungsflüssigkeit eingerichtet ist. Dadurch wird das Risiko, dass sich Rückstände am Wischblatt bilden, verringert.

Wenn die genannte Sprühdüse mit im Rohr befindlichen Kulturmedium (welches die phototrophen Mikroorganismen beinhaltet) gespeist wird, werden üblicherweise auch die Mikroorganismen mit versprüht, wodurch sie hohen Scherkräften ausgesetzt sind. Dies kann zum Absterben von Mikroorganismen (insbesondere einige Algenarten sind höchst empfindlich in Bezug auf Scherkräfte) führen und somit die Ausbeute verringern. Aufgrund dessen ist in einer bevorzugten Ausführungsform die Sprühdüse über eine Leitung (z.B. über eine Wasserleitung) mit einem Flüssigkeitsreservoir außerhalb des Rohres verbunden. Dieses Flüssigkeitsreservoir enthält bevorzugt eine keimreduzierte oder sterile Reinigungsflüssigkeit (selbstredend nicht das genannte Kulturmedium mit den Mikroorganismen), wie z.B. sterilfiltriertes Wasser.

Gemäß einer weiteren, ganz besonders bevorzugten Ausführung der vorliegenden Erfindung ist im Betriebszustand des Photobioreaktors die Instandhaltungsvorrichtung im Rohr für das Kulturmedium umströmbar und/oder durchströmbar (z.B. indem sie in Form eines Hohlzylinders ausgebildet ist). Dadurch kann (auch bei längerer Verweildauer im Rohr) der fortlaufende Betrieb des Photobioreaktors besser gewährleistet werden. Vorzugsweise blockiert die Instandhaltungsvorrichtung höchstens 97,5%, bevorzugt höchstens 95%, mehr bevorzugt höchstens 92,5% oder gar höchstens 90%, noch mehr bevorzugt höchstens 87,5% oder gar höchstens 85%, insbesondere höchstens 82,5% oder gar höchstens 80% der Querschnittsfläche des Rohres an der am stärksten durch die Instandhaltungsvorrichtung blockierten Stelle im Rohr.

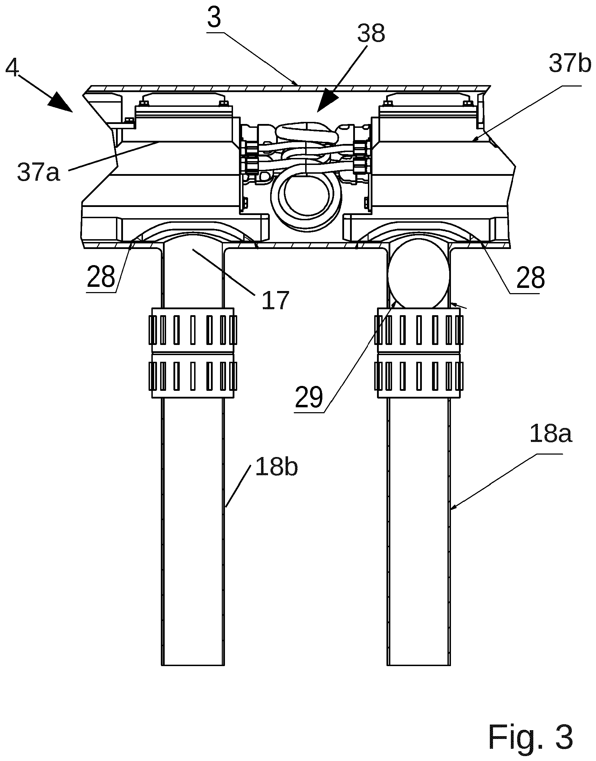

Die Instandhaltungsvorrichtung ist in einer vorteilhaften Weiterbildung derart ausgelegt, dass sie, während sie sich im Rohr, welches einen ersten Durchmesser aufweist, befindet, ein mit dem Rohr in einem Winkel (von bevorzugt zwischen 40° und 140°, mehr bevorzugt zwischen 60° und 120°, noch mehr bevorzugt zwischen 80° und 100°, insbesondere im Wesentlichen 90°) flüssigkeitsdurchlässig verbundenes, weiteres Rohr, welches gegebenenfalls einen Durchmesser kleiner oder größer, vorzugsweise kleiner, dem ersten Durchmesser aufweist, mit einem von der Instandhaltungsvorrichtung freisetzbaren Reinigungsinstrument reinigen kann (vgl. Fig. 3), gegebenenfalls zeitgleich mit der Reinigung des erstgenannten Rohres. Auf diese Weise kann für miteinander verbundene Rohre verschiedenen Durchmessers dieselbe Instandhaltungsvorrichtung eingesetzt werden.

In der erfindungsgemäßen Ausführung des Photobioreaktors ist das Rohr ein Verteilrohr ("Manifold") mit zumindest drei Anschlüssen für den Zufluss bzw. Abfluss von Kulturmedium. Der Photobioreaktor ist in dieser Ausführung derart ausgelegt, dass die Instandhaltungsvorrichtung zu zumindest einem der Anschlüsse im Rohr bewegt werden kann und diesen abdichten kann. So können ohne Störung des laufenden Betriebs (während das Kulturmedium im Rohr weiterströmt) Wartungsarbeiten jenseits des abgedichteten Anschlusses vorgenommen werden. Die genannten Anschlüsse können z.B. als Bohrungen, Öffnungen oder Anschlussfortsätze ausgebildet sein.

Es ist günstig, wenn das Rohr (insbesondere wenn es als Verteilrohr ausgebildet ist) entlang seiner Längsachse kein Gefälle bzw. keine Steigung mit einem Winkel (in Bezug auf den Spiegel des Kulturmediums) von mehr als 10°, bevorzugt von mehr als 5°, mehr bevorzugt von mehr als 2,5°, noch mehr bevorzugt von mehr als 1° oder gar von mehr als 0,5° aufweist. Besonders bevorzugt ist das Verteilrohr (entlang seiner Längsachse) im Wesentlichen horizontal oder horizontal orientiert (d.h. seine Längsachse ist im Wesentlichen parallel bzw. parallel zum Spiegel des Kulturmediums).

Ein besonderer Vorteil der Erfindung ist die genannte Abdichtungsfunktion. Das Reaktorelement weist ferner eine Vielzahl von Steig- und Fallrohren für das flüssiges Kulturmedium auf, wobei die Steig- und Fallrohre jeweils an ihrem oberen Ende an das als Verteilrohr ausgebildete Rohr flüssigkeitsdurchlässig angeschlossen sind, wobei zumindest eines der Steig- und eines der Fallrohre zusätzlich miteinander durch ein Verbindungsstück flüssigkeitsdurchlässig verbunden sind. Dabei ist der Photobioreaktor derart ausgelegt, dass die Instandhaltungsvorrichtung gleichzeitig den Anschluss für das Fallrohr und den Anschluss für das Steigrohr, welche beide miteinander zusätzlich durch ein Verbindungsstück flüssigkeitsdurchlässig verbunden sind (d.h. sozusagen ein "U-Rohr" bilden können), abdichten kann. Dadurch kann das Steigrohr bzw. das Fallrohr bzw. das Verbindungsstück ausgetauscht werden, ohne dass der laufende Betrieb gestört wird. Üblicherweise muss zuvor lediglich das in Steig- und Fallrohr (bzw. im Verbindungsstück) befindliche Kulturmedium abgelassen werden.

Ein Verbindungsstück verbindet vorzugsweise genau eines der Steigrohre mit genau einem der Fallrohre flüssigkeitsdurchlässig (bzw. schließt die beiden aneinander flüssigkeitsdurchlässig an). Bevorzugt ist dabei, dass zumindest alle bis auf ein oder zwei Steigrohre des Reaktorelements und/oder zumindest alle bis auf ein oder zwei Fallrohre des Reaktorelements je paarweise mit einem solchen Verbindungsstück flüssigkeitsdurchlässig verbunden sind, insbesondere sind zumindest alle bis auf ein Steigrohr des Reaktorelements und/oder zumindest alle bis auf ein Fallrohr des Reaktorelements je paarweise mit einem solchen Verbindungsstück flüssigkeitsdurchlässig verbunden. Es ist konstruktiv günstig (insbesondere zur Unterstützung einer mäanderförmigen Strömung), wenn ein Verbindungsstück ein Steigrohr und ein Fallrohr, die zueinander benachbart sind (d.h. benachbart an das Verteilrohr angeschlossen sind), miteinander flüssigkeitsdurchlässig verbindet.

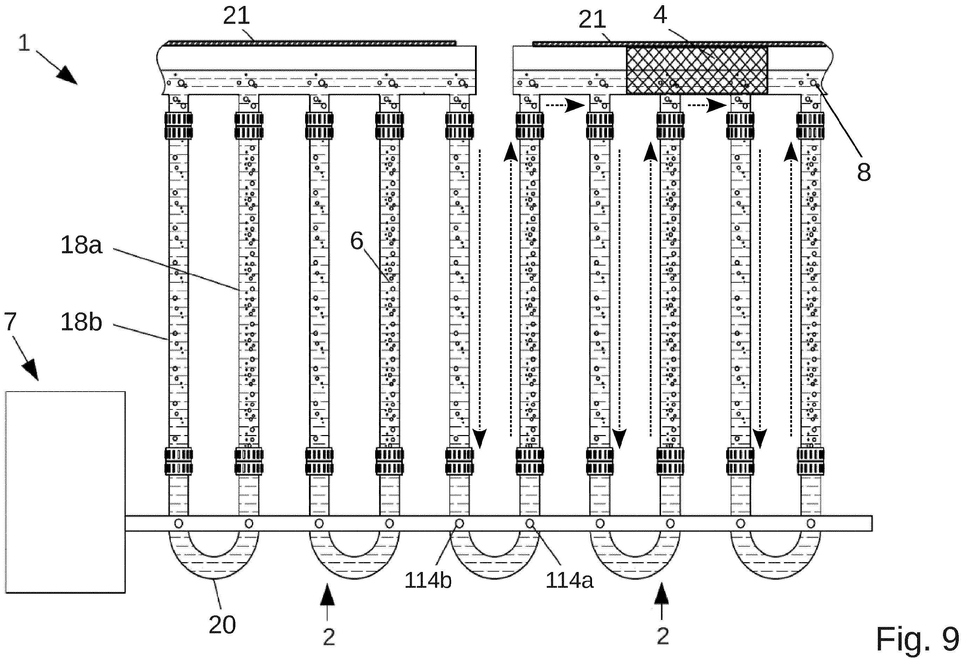

Erfindungsgemäß beträgt die genannte Vielzahl von Steig- bzw. Fallrohren des Reaktorelements, die mit dem selben als Verteilrohr ausgebildeten Rohr flüssigkeitsdurchlässig verbunden sind, zumindest drei (z.B. in der Anordnung Fallrohr - Steigrohr - Fallrohr oder Steigrohr - Fallrohr - Steigrohr entlang des Verteilrohres). Es ist im Sinne der Skalierbarkeit der Reinigung günstig, wenn die genannte Vielzahl zumindest vier, bevorzugt zumindest fünf, mehr bevorzugt zumindest zehn, noch mehr bevorzugt zumindest zwanzig, insbesondere zumindest dreißig oder gar zumindest vierzig oder zumindest fünfzig oder zumindest hundert beträgt. Vorteilhafterweise wird eine alternierende Anordnung von Steig- bzw. Fallrohren entlang des Verteilrohres gewählt (also z.B. Steigrohr - Fallrohr - Steigrohr - ... - Fallrohr - Steigrohr oder Steigrohr - Fallrohr - Steigrohr - ... - Fallrohr - Steigrohr - Fallrohr, oder Fallrohr - Steigrohr - Fallrohr - ... - Steigrohr). Zweckmäßigerweise ist die Positionierung von Ein- bzw. Auslässen (z.B. am oberen oder unteren Ende) im Photobioreaktor natürlich gemäß dieser Anordnung so zu wählen, dass ein Totvolumen vermieden wird. Für den Fachmann ist evident, dass durch Umdrehen des Flusses des Kulturmediums ein Steigrohr zu einem Fallrohr werden kann bzw. umgekehrt. Vorteilhafterweise ergibt sich aus der Summe der Flussgeschwindigkeitsvektoren der im Verteilrohr herrschenden Strömungen des Kulturmediums (insbesondere wenn im Reaktorelement eine mäanderförmige Strömung geführt wird) ein Flussgeschwindigkeitsvektor, der im Wesentlichen parallel zur Längsachse des Verteilrohres ist (sozusagen eine Netto-Strömung entlang der Längsachse, vgl. auch die horizontalen gestrichelten Pfeile in Fig. 9).

Um die Reinigung zu erleichtern, weisen das Rohr (insbesondere wenn es als Verteilrohr ausgebildet ist) und/oder die Steig- bzw. Fallrohre zweckmäßigerweise, gegebenenfalls unabhängig voneinander, entlang ihrer Längsachse keine Biegung von mehr als 90°, bevorzugt von mehr als 70°, mehr bevorzugt von mehr als 50°, noch mehr bevorzugt von mehr als 30° oder gar von mehr als 20°, insbesondere von mehr als 10° oder gar von mehr als 5° auf. Es ist besonders günstig, wenn das Rohr (insbesondere wenn es als Verteilrohr ausgebildet ist) und/oder die Steig- bzw. Fallrohre entlang ihrer Längsachse im Wesentlichen gerade oder gerade sind. Bevorzugt haben das Rohr (insbesondere wenn es als Verteilrohr ausgebildet ist) und/oder die Steig- bzw. Fallrohre (gegebenenfalls bis auf etwaige Anschlussfortsätze) ein im Wesentlichen rundes Profil.

In einer weiteren, ganz besonders bevorzugten Ausführungsform ist die Instandhaltungsvorrichtung dazu eingerichtet, das durch die Instandhaltungsvorrichtung gegenüber dem Rohr abgedichtete Fallrohr und das durch die Instandhaltungsvorrichtung gegenüber dem Rohr abgedichtete Steigrohr, welche beide miteinander durch ein Verbindungsstück flüssigkeitsdurchlässig verbunden sind (d.h. z.B. ein "U-Rohr" bilden können), zu reinigen. Vorzugsweise wird dafür ein von der Instandhaltungsvorrichtung freisetzbares Reinigungsinstrument wie z.B. ein Schwamm freigesetzt, durch das genannte Fallrohr und das genannte Steigrohr (welche z.B. ein "U-Rohr" bilden) gepumpt (auch daher weist die Instandhaltungsvorrichtung bevorzugt eine Pumpe, bevorzugt eine Membranpumpe, auf), und zweckmäßigerweise wieder von der Instandhaltungsvorrichtung aufgenommen (sodass es zur nächsten Freisetzung wieder zur Verfügung steht). Auf diese Weise können Teilstücke des Reaktorelements intensiver gereinigt werden, während der Betrieb des verbleibenden Teils des Photobioreaktors möglichst ungestört bleibt.

Es ist günstig, wenn die Längsachsen der Steig- bzw. Fallrohre, gegebenenfalls unabhängig voneinander, in einem Winkel von mehr als 5°, bevorzugt von mehr als 20°, mehr bevorzugt von mehr als 40° oder gar von mehr als 60°, noch mehr bevorzugt von mehr als 70° oder gar von mehr als 80°, insbesondere von mehr als 85° oder gar von mehr als 87,5° zum Spiegel des Kulturmediums bzw. zum als Verteilrohr ausgebildeten Rohr stehen. Besonders günstig ist, wenn die Längsachsen der Steig- bzw. Fallrohre im Wesentlichen normal (bzw. normal) zum Spiegel des Kulturmediums bzw. zum (bevorzugt im Wesentlichen horizontalen) Verteilrohr stehen (mit anderen Worten: wenn diese vertikal orientiert sind). Zweckmäßigerweise sind die Längsachsen der Steig- und Fallrohre (insbesondere derer, die jeweils durch ein Verbindungsstück verbunden sind) zueinander im Wesentlichen parallel.

Der Photobioreaktor der vorliegenden Erfindung ist vorzugsweise vom Typ her ein Rohrreaktor ("tubular reactor"), genauer gesagt ein vertikaler Rohrreaktor ("vertical tubular reactor"), bzw. kein Plattenreaktor ("plate reactor" bzw. "flat panel reactor").

Gerade bei größeren Photobioreaktoren (die z.B. mit einem Reaktorelement mit langen Verteilrohr ausgestattet sind, an dem dutzende oder hunderte von Steig- bzw. Fallrohren angeschlossen sind) kann sich das Problem ergeben, dass sich die Instandhaltungsvorrichtung über die Länge unerwünscht verdreht bzw. stellt es eine technische Herausforderung dar, die Instandhaltungsvorrichtung präzise an die richtige Stelle im Rohr zu steuern (d.h. z.B. das richtige U-Rohr präzise zu erreichen). Infolgedessen ist in einer weiteren bevorzugten Ausführungsform der vorliegenden Erfindung am Rohr, bevorzugt an dessen Außenseite, eine Führungsleiste zur Stabilisierung und/oder Positionierung der Instandhaltungsvorrichtung im Rohr vorgesehen. Beispielsweise kann die Führungsleiste zur Stabilisierung magnetisch sein und die Instandhaltungsvorrichtung einen Magneten aufweisen (oder umgekehrt). Zur Positionierung der Instandhaltungsvorrichtung kann die Führungsleiste beispielsweise an bestimmten Stellen an der Führungsleiste Kodierungen, z.B. magnetische Codeträger wie Magnetstreifen oder optische Kodierungen (z.B. Bar-Codes), aufweisen, die von der Instandhaltungsvorrichtung abgelesen werden können, woraufhin, wenn die anzusteuernde Position erreicht ist, die Instandhaltungsvorrichtung abgebremst wird.

In einer weiteren, bevorzugten Ausführungsform der vorliegenden Erfindung weist das genannte Antriebssystem zumindest eine Seilwinde (bevorzugt zumindest zwei Seilwinden an gegenüberliegenden Enden des Rohres), die ein im Rohr geführtes und mit der Instandhaltungsvorrichtung verbundenes Seil antreiben kann, auf. Dies hat sich als effiziente und robuste Antriebsweise für die Instandhaltungsvorrichtung herausgestellt. Über Leitungen im Seil kann die Instandhaltungsvorrichtung z.B. mit Strom, Druckluft bzw. Reinigungsflüssigkeit (z.B. über eine Hochdruckleitung) versorgt werden oder Steuersignale empfangen. Es ist von Vorteil, wenn die Seilwinde mit einer, insbesondere zwischen Seilwinde und Rohr geschalteten, Einrichtung zur Desinfektion des (gegebenenfalls darin aufgerollten) Seiles ausgestattet ist (z.B. Abstreifer für das Seil am Deckelaustritt der Seilwinde - Sprühdüsen mit EtOH oder Desinfektionslösung - Abstreifer für das Seil nach Besprühung). Das Seil ist nämlich, wenn es aus dem Rohr gezogen wird, üblicherweise feucht und stellt so einen möglichen Nährboden für unerwünschte Mikroorganismen dar (insbesondere wenn es aufgerollt über längere Zeit in der Seilwinde vorliegt). Diese kontaminierenden Mikroorganismen können in den Photobioreaktor eingeschleppt werden, wenn das Seil aus der Seilwinde wieder in das Rohr gezogen wird (z.B. wenn die Instandhaltungsvorrichtung von einer zweiten Seilwinde in die entgegengesetzte Richtung gezogen wird). Durch eine Einrichtung zur Desinfektion des Seiles werden solche Kontaminationen jedoch zumindest weitgehend vermieden. Darüber hinaus oder alternativ dazu kann die Einrichtung zur Desinfektion beispielsweise eine Ozonbegasungsanlage für den Windenraum aufweisen.

Um das Kontaminationsrisiko durch das Seil (zusätzlich oder alternativ dazu, dass eine Einrichtung zur Desinfektion des Seiles z.B. wie oben beschrieben vorgesehen ist) zu verringern, ist in einer weiteren bevorzugten Ausführungsform vorgesehen, dass das im Rohr geführte und mit der Instandhaltungsvorrichtung verbundene Seil im Rohr zumindest teilweise oberhalb des Spiegels des Kulturmediums (bzw. zumindest teilweise in besagtem Gasraum des Verteilrohrs) geführt wird. Dadurch wird der Kontakt des Seiles mit dem Kulturmedium reduziert.

Im Allgemeinen sollen Rohre für Photobioreaktoren chemisch widerstandsfähig sein und nicht anfällig für Anlagerungen von Rückständen bzw. für (UV-bedingte) Trübungen, welche die Lichteinstrahlung verringern und damit die Ausbeute schmälern können. Allerdings hat sich im Zuge der vorliegenden Erfindung herausgestellt, dass es technisch äußerst aufwändig oder gar unmöglich wäre, solche Rohre für Photobioreaktoren mit geringen Fertigungstoleranz herzustellen, insbesondere wenn sie im Wesentlichen aus Glas sind (welches ansonsten hervorragende Eigenschaften für den Einsatz im Photobioreaktor hätte). Folglich besteht das Risiko, dass eine Instandhaltungsvorrichtung, insbesondere wenn sie eine gewisse Länge überschreitet (die jedoch erforderlich sein kann, um alle gewünschten Funktionalitäten in der Instandhaltungsvorrichtung unterzubringen) und rigide ist, im Rohr stecken bleibt. Daher weist die Instandhaltungsvorrichtung zweckmäßigerweise zumindest zwei Module ("Waggons") auf, die durch eine Kupplung miteinander verbunden sind, um das genannte Risiko zu verringern und sozusagen eine gewisse Flexibilität hinsichtlich der Fertigungstoleranz sicherzustellen.

Um das Risiko, dass sich an der Instandhaltungsvorrichtung Verschmutzungen ablagern (die wie eingangs erläutert eine ständige Gefahr hinsichtlich Kontaminationen der Kultur darstellen), weiter zu reduzieren, weist der Photobioreaktor gemäß einer vorteilhaften Weiterbildung der Erfindung eine Station ("Bahnhof") für die Instandhaltungsvorrichtung auf, die zur Reinigung der Instandhaltungsvorrichtung und/oder zur Entnahme (bzw. zum Einsetzen) der Instandhaltungsvorrichtung aus dem Photobioreaktor (bzw. in den Photobioreaktor) eingerichtet ist und in welche die Instandhaltungsvorrichtung bewegt werden kann. Vorzugsweise kann diese Station von der Instandhaltungsvorrichtung direkt aus dem Rohr befahren werden. Es ist besonders bevorzugt, wenn diese Station mit zumindest einer Sprühdüse zur Reinigung der Instandhaltungsvorrichtung ausgestattet ist, wobei die Sprühdüse vorzugsweise über eine Leitung mit einem Flüssigkeitsreservoir außerhalb des Rohres verbunden ist.

Um das Risiko, dass sich an der Instandhaltungsvorrichtung Verschmutzungen ablagern, noch weiter zu verringern, weist das Rohr in einer bevorzugten Ausführungsform der vorliegenden Erfindung eine Einrichtung zur Reinigung der Instandhaltungsvorrichtung auf. Diese Einrichtung umfasst vorzugsweise zumindest eine Sprühdüse, insbesondere zumindest eine ringförmige Anordnung von Sprühdüsen durch welche die Instandhaltungsvorrichtung zur Reinigung bewegt werden kann. Vorzugsweise ist diese Sprühdüse bzw. sind die Sprühdüsen der Anordnung über eine Leitung mit einem Flüssigkeitsreservoir außerhalb des Rohres verbunden.

Die vorliegende Erfindung ermöglicht in der Regel, dass statt Einweg-Kunststoffkomponenten (hochwertigere) Glasbauteile verwendet werden können, unter anderem auch weil das Risiko für Glasbruch sinkt, wenn eine Demontage zur Reinigung weniger oft erforderlich ist. Somit bestehen in einer bevorzugten Ausführungsform des Photobioreaktors das genannte Rohr und/oder die genannten Steig- und Fallrohre im Wesentlichen aus Glas. Dadurch wird unter anderem die Langlebigkeit bzw. Widerstandsfähigkeit des Photobioreaktors erhöht, insbesondere wenn Mikroalgen kultiviert werden, die ein Kulturmedium mit extremem pH-Wert benötigen. Hinzu kommt, dass sich Glas typischerweise mit der Zeit weniger aufraut als der üblicherweise verwendete Kunststoff, so dass die Reinigung bzw. das Sauberhalten vereinfacht wird. Das Glas kann beschichtet sein, wie beispielsweise in der DE 10 2009 029 792 A1 oder der WO 2011/015653 A2 offenbart.

In einer weiteren, bevorzugten Ausführung wird die innere Oberfläche des Rohres bzw. die Fall- und Steigrohre, wenn vorhanden, von der Instandhaltungsvorrichtung zu einem voreinstellbaren Zeitpunkt gereinigt, vorzugsweise wobei dieser Vorgang zu zumindest einem weiteren voreinstellbaren Zeitpunkt wiederholt wird.

Gemäß einer weiteren bevorzugten Ausführungsform ist die Instandhaltungsvorrichtung dazu eingerichtet, im Rohr eines oder mehrere der folgenden in das Kulturmedium einzubringen (bevorzugt an einer voreinstellbaren Stelle im Rohr gegebenenfalls zu einem voreinstellbaren Zeitpunkt und/oder abhängig von einem (im Folgenden beschriebenen) Sensor bestimmten Messwert): Nährstoffe, Hilfsstoffe, weitere phototrophe Mikroorganismen bzw. weiteres Kulturmedium. Alternativ oder zusätzlich weist die Instandhaltungsvorrichtung gemäß einer weiteren bevorzugten Ausführungsform des erfindungsgemäßen Photobioreaktors einen Sensor zum Bestimmen einer Konzentration eines Stoffes im Kulturmedium (z.B. ein pH-Meter) bzw. der Mikroorganismen-Konzentration im Kulturmedium (z.B. ein Photometer) bzw. der Temperatur auf, bevorzugt wobei die Bestimmung an einer voreinstellbaren Stelle im Rohr gegebenenfalls zu einem voreinstellbaren Zeitpunkt durchgeführt werden kann.

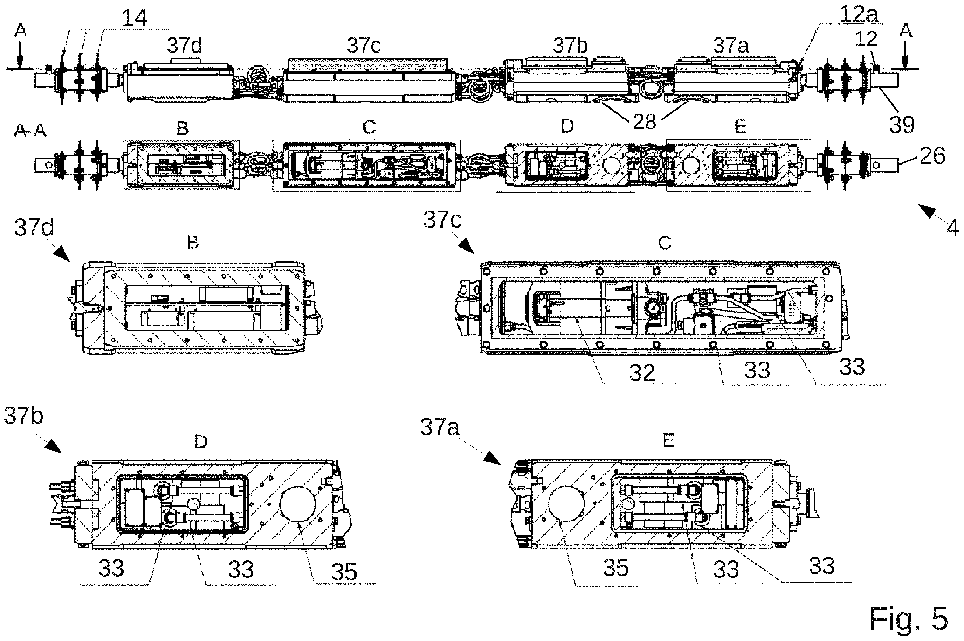

Üblicherweise besteht ein Teil der Oberfläche der Instandhaltungsvorrichtung, welche Oberfläche dazu vorgesehen ist, mit dem Kulturmedium in Kontakt zu kommen, aus Kunststoff (z.B. in Form von Polyethylen-Schläuchen zwischen Modulen der Instandhaltungsvorrichtung, deren Außenoberfläche mit dem Kulturmedium in Kontakt kommt; vgl. Fig. 5). Da im Photobioreaktor bevorzugt phototrophe Mikroorganismen zur Lebensmittelproduktion kultiviert werden, ist es aus Gründen der Lebensmittelsicherheit zweckmäßig, wenn dieser Teil den Gesamtmigrationsgrenzwert von 10 mg/dm² nicht überschreitet.

In einem weiteren Aspekt stellt die vorliegende Erfindung eine bewegliche Instandhaltungsvorrichtung für die Instandhaltung der inneren Oberfläche eines Rohres eines Photobioreaktors zur Kultivierung phototropher Mikroorganismen zur Verfügung. Diese ist für die Instandhaltung der inneren Oberfläche des Rohres im laufenden Betrieb des Photobioreaktors, während das Rohr von flüssigem Kulturmedium mit den Mikroorganismen zumindest teilweise durchströmt wird, geeignet, und dafür eingerichtet, von einem externen Antriebssystem, welches bevorzugt eine Seilwinde aufweist, zumindest gegen die Strömung des Kulturmediums im Rohr bewegt zu werden. Auch dadurch wird die eingangs genannte Aufgabe gelöst.

Zweckmäßigerweise weist die bewegliche Instandhaltungsvorrichtung ein Verbindungsteil zur Verbindung mit dem externen Antriebssystem auf. Bevorzugt ist dies ein Mittel zur Verankerung eines Seiles (z.B. eine Öse), um von einer Seilwinde zumindest gegen die Strömung des Kulturmediums bewegt werden zu können.

In einer bevorzugten Ausführung weist die bewegliche Instandhaltungsvorrichtung eine Sprühdüse zum Besprühen der inneren Oberfläche des Rohres mit einer Reinigungsflüssigkeit auf. Dies hat sich im Dauerbetrieb als schonender und hygienischer im Vergleich zu einer Ausstattung mit Bürsten erwiesen. Zur Versorgung der Sprühdüse mit der Reinigungsflüssigkeit weist die Instandhaltungsvorrichtung einen Anschluss für eine Leitung mit der Reinigungsflüssigkeit auf, denn aus den weiter oben diskutierten Gründen kann das Versprühen des Kulturmediums mit den Mikroorganismen selbst nachteilig sein.

In einer weiteren bevorzugten Ausführungsform weist die bewegliche Instandhaltungsvorrichtung zumindest ein Wischblatt aus einem elastischen Material wie beispielsweise einem Kautschuk zum Wischen der inneren Oberfläche des Rohres auf. Auch dies hat sich im Dauerbetrieb als schonender und hygienischer im Vergleich zu einer Ausstattung mit Bürsten erwiesen. Besonders bevorzugt ist dabei, wenn die Instandhaltungsvorrichtung zumindest zum Wischen von mit dem genannten Gasraum in Berührung befindlicher innerer Oberfläche des Rohres mit dem Wischblatt eingerichtet ist. Auf diese Weise können Ablagerungen (die sich wie oben geschildert im Laufe der Zeit ergeben können) besser beseitigt werden. Alternativ, oder zusätzlich, weist die Instandhaltungsvorrichtung bevorzugt eine, gegebenenfalls weitere, Sprühdüse auf, die zum Besprühen des Wischblatts mit einer Reinigungsflüssigkeit eingerichtet ist. Dadurch wird das Risiko, dass sich eingetrocknete Rückstände am Wischblatt bilden, verringert.

Des Weiteren weist die Instandhaltungsvorrichtung zweckmäßigerweise zumindest zwei Module ("Waggons") auf, die durch eine Kupplung miteinander verbunden sind, um sozusagen eine gewisse Flexibilität hinsichtlich der Fertigungstoleranz des Rohres sicherzustellen.

Die bewegliche Instandhaltungsvorrichtung ist ferner mit einer Einrichtung zum Abdichten von Anschlüssen des Rohres ausgestattet. Diese Einrichtung umfasst bevorzugt eine elastische Dichtung (z.B. einen aufblasbaren Dichtungsring) mit Hohlraum, in den ein Fluid (z.B. Druckluft oder die genannte Reinigungsflüssigkeit) druckbeaufschlagt eingebracht werden kann, um die Dichtung auszudehnen und dadurch den Anschluss zu verschließen. Bevorzugt weist die Instandhaltungsvorrichtung eine Pumpe, insbesondere eine Membranpumpe, zur Einbringung des Fluids in den Hohlraum auf.

Üblicherweise besteht ein Teil der Außenoberfläche der beweglichen Instandhaltungsvorrichtung aus Kunststoff (z.B. in Form von Polyethylen-Schläuchen zwischen Modulen der Instandhaltungsvorrichtung, deren Außenoberfläche mit dem Kulturmedium in Kontakt kommt; vgl. Fig. 5). Da im Photobioreaktor bevorzugt phototrophe Mikroorganismen zur Lebensmittelproduktion kultiviert werden, ist es aus Gründen der Lebensmittelsicherheit zweckmäßig, wenn dieser Teil den Gesamtmigrationsgrenzwert von 10 mg/dm² nicht überschreitet.

Die soeben beschriebene bewegliche Instandhaltungsvorrichtung kann zur Instandhaltung der inneren Oberfläche eines Rohres eines Photobioreaktors zur Kultivierung phototropher Mikroorganismen verwendet werden, wobei die Instandhaltung im laufenden Betrieb des Photobioreaktors, vorzugsweise während das Kulturmedium Sonnenlicht oder Kunstlicht ausgesetzt wird, wobei das Rohr von flüssigem Kulturmedium mit den Mikroorganismen zumindest teilweise durchströmt wird, erfolgt.

Unter "Betriebszustand" ist hierin ein Zustand des Photobioreaktors zu verstehen, in dem lebende phototrophe Mikroorganismen in Kulturmedium im Photobioreaktor (insbesondere im Rohr) kultiviert werden, wobei das Kulturmedium im Photobioreaktor (insbesondere im Rohr) vorzugsweise eine Strömung aufweist.

Hierin wird unter Gesamtmigrationsgrenzwert ("overall migration limit") die höchstzulässige Menge nichtflüchtiger Stoffe, die aus einem Material oder Gegenstand in Lebensmittelsimulanzien abgegeben werden, verstanden. Bevorzugt wird dieser Gesamtmigrationsgrenzwert ermittelt nach der Verordnung (EU) Nr. 10/2011 der Kommission vom 14. Januar 2011 über Materialien und Gegenstände aus Kunststoff, die dazu bestimmt sind, mit Lebensmitteln in Berührung zu kommen, in der konsolidierten Fassung vom 26. Februar 2015 (CELEX-Nr. 02011R0010-20150226; im weiteren genannt "die Verordnung"), welche bereits die folgenden Änderungen umfasst: geändert durch Durchführungsverordnung (EU) Nr. 321/2011 der Kommission vom 1. April 2011, Verordnung (EU) Nr. 1282/2011 der Kommission vom 28. November 2011, Verordnung (EU) Nr. 1183/2012 der Kommission vom 30. November 2012, Verordnung (EU) Nr. 202/2014 der Kommission vom 3. März 2014, Verordnung (EU) Nr. 865/2014 der Kommission vom 8. August 2014, Verordnung (EU) 2015/174 der Kommission vom 5. Februar 2015; und berichtigt durch Berichtigung, Amtsblatt L 278 vom 25. Oktober 2011, S. 13 (10/2011). Insbesondere werden dafür im Kontext der vorliegenden Erfindung die Standardprüfungsbedingungen "OM 2" gemäß Anhang V, Kapitel 3 der Verordnung, mit einem Lebensmittelsimulanz, ausgewählt aus den Lebensmittelsimulanzien A, B, C, D1 und D2 gemäß Anhang III der Verordnung, bevorzugt ausgewählt aus A, B und C, noch mehr bevorzugt ausgewählt aus A und B, insbesondere A, herangezogen.

Die Erfindung wird nachfolgend anhand von besonders bevorzugten Ausführungsbeispielen, auf die sie jedoch nicht eingeschränkt ist, und unter Bezugnahme auf Zeichnungen noch weiter erläutert. Die Zeichnungen zeigen im Einzelnen:
**Fig. 1** zeigt eine Draufsicht auf eine Ausführung des erfindungsgemäßen Photobioreaktors im Betriebszustand;
**Fig. 2** zeigt eine Draufsicht auf eine Ausführung des erfindungsgemäßen Photobioreaktors, in der die Instandhaltungsvorrichtung detaillierter dargestellt ist;
**Fig. 3** ist eine lokal vergrößerte Ansicht der Ausführung gemäß Fig. 2, wobei die Abdichtungs- und Reinigungsfunktion der Instandhaltungsvorrichtung für ein miteinander verbundenes Paar eines Steig- und Fallrohres gezeigt wird;
**Fig. 4** zeigt schematisch eine Ausführung des erfindungsgemä-ßen Photobioreaktors mit der Instandhaltungsvorrichtung in der Station außerhalb des Rohres;
**Fig. 5** zeigt verschiedene Ansichten einer bevorzugten Ausführung der erfindungsgemäßen Instandhaltungsvorrichtung;
**Fig. 6** zeigt perspektivische Ansichten einer Instandhaltungsvorrichtung gemäß Fig. 5;
**Fig. 7** zeigt beispielhaft Querschnitte von Modulen der erfindungsgemäßen Instandhaltungsvorrichtung im Rohr, welche die Umströmbarkeit sicherstellen;
**Fig. 8** zeigt eine Draufsicht auf eine Ausführung des erfindungsgemäßen Photobioreaktors im Betriebszustand (Instandhaltungsvorrichtung und Antriebssystem sind in dieser Ansicht nicht gezeigt);
**Fig. 9** zeigt eine Draufsicht auf eine Ausführung des erfindungsgemäßen Photobioreaktors im Betriebszustand;
**Fig. 10** ist eine lokal vergrößerte Ansicht der Ausführung gemäß Fig. 8 bzw. Fig. 9;
**Fig. 11A** zeigt eine Draufsicht auf ein befülltes Rohr, das als Verteilrohr ausgebildet ist, wie es für die vorliegenden Erfindung verwendet werden kann;
**Fig. 11B** zeigt eine perspektivische Ansicht des Rohres gemäß Fig. 11A;
**Fig. 12** zeigt eine perspektivische Ansicht eines Metallgerüsts, wie es für Ausführungen von Reaktorelementen des erfindungsgemäßen Photobioreaktors werden kann;
**Fig. 13** zeigt eine perspektivische Ansicht von Reaktorelementen einer Ausführungsform des erfindungsgemäßen Photobioreaktors.

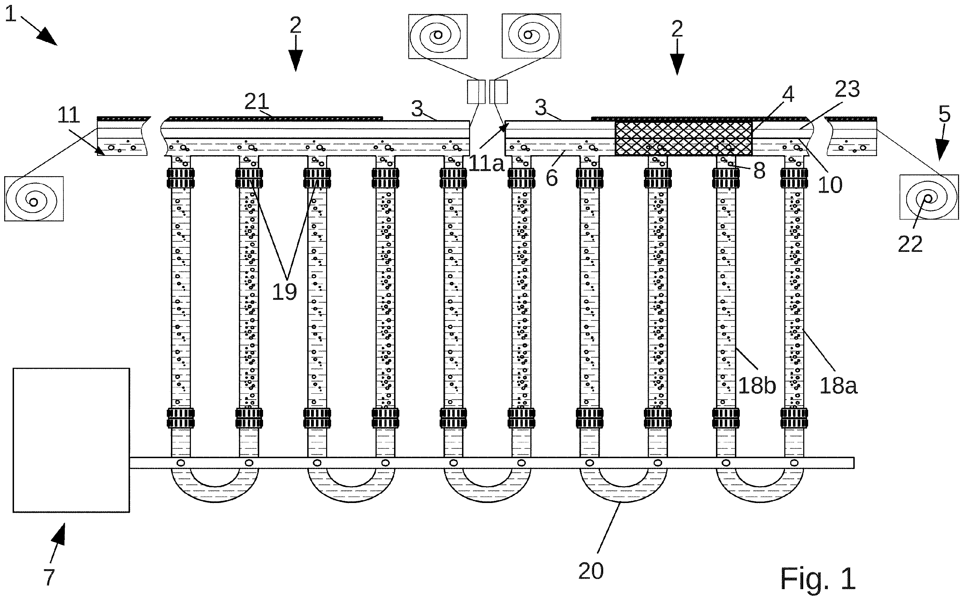

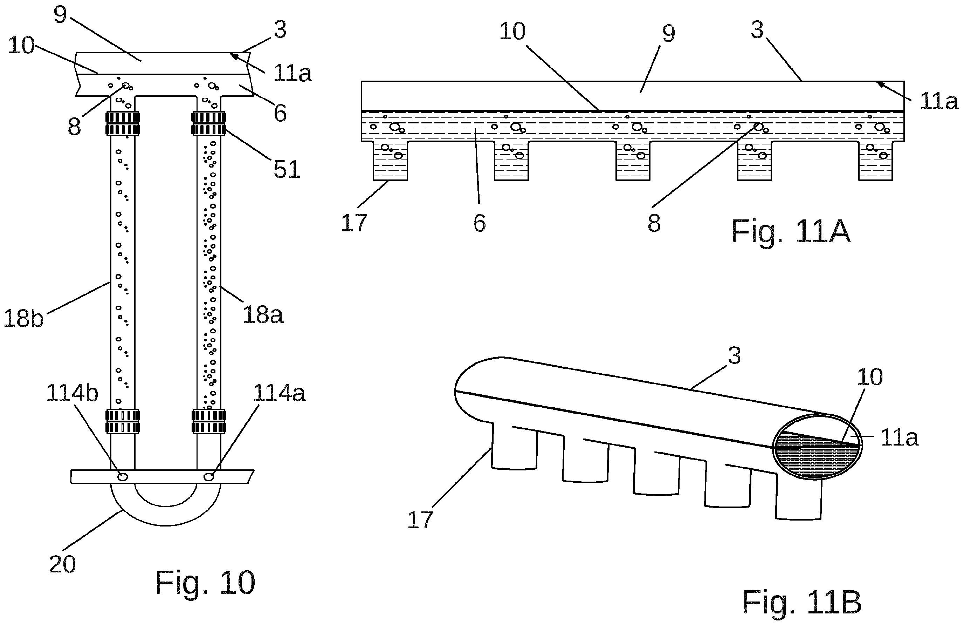

Fig. 1 zeigt einen Photobioreaktor 1 zur Kultivierung von phototrophen Mikroorganismen, mit zwei Reaktorelementen 2, die jeweils eine Vielzahl von vertikalen, geraden Steigrohren 18a und Fallrohren 18b für Kulturmedium 6 und ein Rohr 3, welches horizontal, gerade und als Verteilrohr ausgebildet ist und ferner ein kreisförmiges Profil hat, aufweisen. Das Kulturmedium 6 ist ein flüssiges Kulturmedium auf Wasserbasis und angereichert mit Nährstoffen; es beinhaltet die phototrophen Mikroorganismen (üblicherweise Mikroalgen). Das Rohr 3 und die Steig- bzw. Fallrohre 18a bzw. 18b bestehen aus Glas. Die Steigrohre 18a und Fallrohre 18b sind jeweils mit dem als Verteilrohr ausgebildeten Rohr 3 flüssigkeitsdurchlässig verbunden. Die Steigrohre 18a und die Fallrohre 18b sind zusätzlich miteinander durch je ein U-förmiges Verbindungsstück 20 flüssigkeitsdurchlässig verbunden (vgl. auch Fig. 10).

Die Instandhaltungsvorrichtung 4 ist zur Reinigung der inneren Oberfläche des Rohres 3 eingerichtet, indem sie mit Sprühdüsen und Wischblättern für diese Oberfläche ausgestattet ist. Sie weist einen Magneten auf und wird daher durch die magnetische Führungsleiste 21, die an der Außenseite des Rohres 3 angebracht ist, im Rohr 3 stabilisiert. Folglich wird verhindert, dass sich die Instandhaltungsvorrichtung in Bezug auf die Längsachse des Rohres 3 verdrehen kann, wenn sie vom Antriebsystem 5, welches Seilwinden 22 aufweist, unter Verwendung der Seile 23 durch das Rohr 3 gezogen wird. Die Führungsleiste 21 weist ferner Kodierungen auf. Diese markieren die Stellen im Rohr 3, an denen die einzelnen Steig- bzw. Fallrohre 18a bzw. 18b angeschlossen sind, und können von der Instandhaltungsvorrichtung 4 abgelesen und einem Steuerungssystem übermittelt und von diesem verarbeitet werden.

Der Photobioreaktor 1 weist ferner eine Einrichtung 7 zur Begasung des Kulturmediums 6 mit Kohlendioxid auf, wobei das Gas in die Steigrohre 18a bzw. die Fallrohre 18b eingeleitet wird. Das Kulturmedium 6 mit den phototrophen Mikroorganismen steht in den Reaktorelementen 2 so hoch, dass auch die als Verteilrohre ausgebildeten Rohre 3 jeweils etwa zumindest zu 5%, bevorzugt zumindest zu 10%, mehr bevorzugt zumindest zu 20%, noch mehr bevorzugt zumindest zu 30% oder gar zu 40%, insbesondere zur Hälfte damit befüllt sind. Im Rohr 3 sind also sowohl Kulturmedium 6 als auch, oberhalb des Kulturmediums 6, ein Gasraum 9 zur Aufnahme von aus dem Kulturmedium 6 aufsteigenden Gasblasen 8 vorhanden, wobei im Rohr 3 eine Grenzfläche 10 zwischen dem Kulturmedium 6 und dem Gasraum 9 angeordnet ist. Durch ihren Auftrieb allmählich im Kulturmedium 6 aufsteigende Gasblasen 8 kommen mit der Instandhaltungsvorrichtung 4 in Kontakt und verlangsamen bzw. verhindern das Ansetzen von Rückständen auf Oberflächen der Instandhaltungsvorrichtung 4.

Die Instandhaltungsvorrichtung 4 ist aufgrund ihrer Form vom Kulturmedium 6 (und den darin befindlichen phototrophen Mikroorganismen) umströmbar, so dass sie während des gesamten Betriebes des Photobioreaktors 1 im Rohr 3 verbleiben kann. Im Dauerbetrieb wird sie im Rohr 3 hin- und hergezogen, um durch ihre Reinigungsfunktion das Festsetzen von Verschmutzungen im Rohr 3 (im Speziellen im Bereich der Innenoberfläche des Rohres 3, welcher der Grenzfläche 10 nahe ist bzw. mit ihr in Kontakt steht, und der dadurch in dieser Hinsicht besonders gefährdet ist) bereits in frühen Stadien zu unterbinden. Zwei Sprühdüsen der Instandhaltungsvorrichtung 4 sind so orientiert, dass die Instandhaltungsvorrichtung 4 zumindest zur Reinigung von mit dem Gasraum 9 in Berührung befindlicher innerer Oberfläche 11a des Rohres 3 eingerichtet ist. Auch diese innere Oberfläche 11a ist besonders anfällig für Verschmutzungen, weil Spritzer des Kulturmediums 6 hier leicht trocknen und somit Rückstände wie ausgetrocknete Mikroorganismen hinterlassen können. Dadurch, dass sich die Instandhaltungsvorrichtung 4 wie oben beschrieben durch die Führungsleiste 21 nicht verdrehen kann, ist sichergestellt, dass die Orientierung der genannten Sprühdüsen in Richtung der Oberfläche 11a erhalten bleibt.

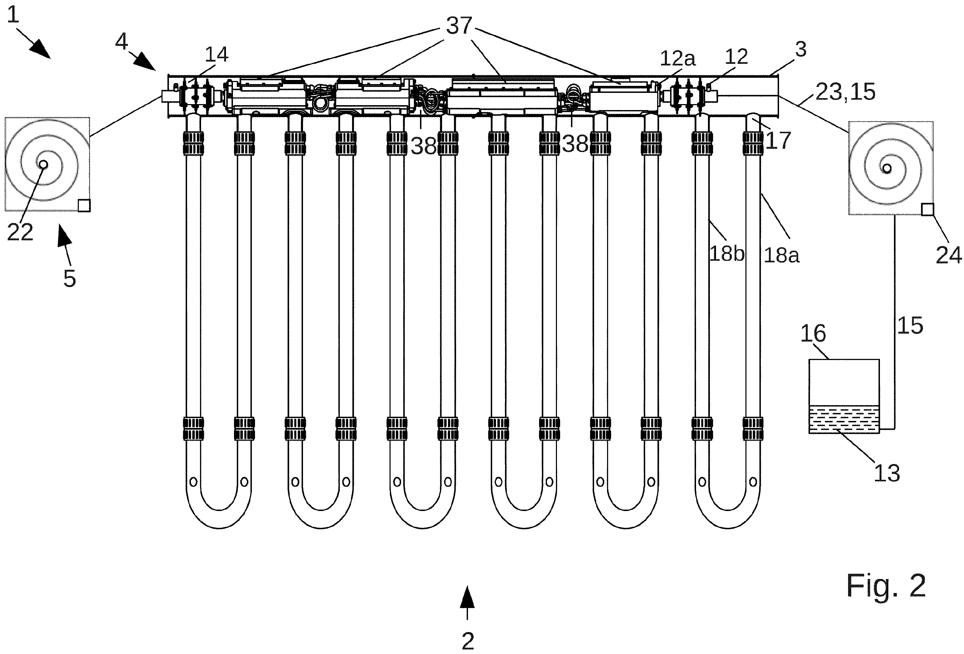

Fig. 2 zeigt einen Photobioreaktor 1 zur Kultivierung von phototrophen Mikroorganismen, mit einem Reaktorelement 2, das eine Vielzahl von vertikalen, geraden Steigrohren 18a und Fallrohren 18b für Kulturmedium 6 und ein Rohr 3, welches horizontal, gerade und als Verteilrohr ausgebildet ist, aufweist. Das Kulturmedium 6 ist ein flüssiges Kulturmedium auf Wasserbasis und angereichert mit Nährstoffen; es beinhaltet die phototrophen Mikroorganismen (üblicherweise Mikroalgen). Die Steigrohre 18a und Fallrohre 18b sind jeweils mit dem als Verteilrohr ausgebildeten Rohr 3 flüssigkeitsdurchlässig verbunden.

Die im Rohr 3 befindliche Instandhaltungsvorrichtung 4 weist vier Module 37 auf, die über Kupplungen 38 miteinander verbunden sind, um fertigungsbedingten Winkelversatz des Rohres 3 auszugleichen, damit die Instandhaltungsvorrichtung 4 nicht im Rohr 3 stecken bleiben kann, wenn sie vom Antriebssystem 5 mit Hilfe der mit dem Seil 23 funktional verbundenen Seilwinden 22 durch das Rohr 3 gezogen wird. Die Seilwinden 22 sind jeweils mit Einrichtungen 24 zur Desinfektion des Seiles 23 ausgestattet, wobei diese Einrichtungen 24 optional als Ozonbegasungsanlagen ausgestaltet sind.

Zur Reinigung der Innenoberfläche Rohres 3 weist die Instandhaltungsvorrichtung 4 die Wischblätter 14 und die Sprühdüsen 12 auf. Die Sprühdüsen 12a dienen dazu, die Wischblätter 14 zu besprühen, um sie feucht zu halten. Dadurch wird zum einen ihre Reinigungswirkung erhöht und zum anderen werden Ablagerungen an den Wischblättern selbst unterbunden. Die Sprühdüsen 12 bzw. 12a werden über eine im Seil 23 vorhandene Leitung 15 aus einem externen Reservoir 16 mit der Reinigungsflüssigkeit 13 (in diesem Fall steriles Wasser) gespeist.

Die Instandhaltungsvorrichtung kann zu jedem einzelnen, miteinander verbundenen Paar von Steig- und Fallrohren 18a bzw. 18b bewegt werden und das Rohr 3 gegenüber diesen abdichten, indem es die entsprechenden Anschlüsse 17 blockiert. Dadurch kann ein Paar von Steig- und Fallrohren 18a bzw. 18b ausgetauscht werden (wobei das Kulturmedium 6 nur aus diesem Teil abgelassen wird), ohne den Betrieb im Rest des Reaktorelements 2 zu stören. Auch eine rasche Abdichtung bei Glasbruch in dem Paar ist so möglich, wobei eine Leckage des Kulturmediums signifikant vermindert wird. Auf diese Weise wird die Instandhaltung des Photobioreaktors wesentlich vereinfacht, insbesondere bei großen Zuchtstrecken mit dutzenden oder hunderten der genannten Paare.

Fig. 3 zeigt die Abdichtungs- und Reinigungsfunktion der Instandhaltungsvorrichtung 4 für ein miteinander verbundenes Paar von Steig- und Fallrohren 18a bzw. 18b, welches an das Rohr 3 über die Anschlüsse 17 angeschlossen ist, im Detail. Die Instandhaltungsvorrichtung 4 weist zwei über die Kupplung 38 miteinander verbundene, im Wesentlichen baugleiche, jedoch entgegengesetzt orientierte Module 37a und 37b auf. Die Module 37a bzw. 37b sind mit je einer Einrichtung 28 zum Abdichten von Anschlüssen des Rohres 3 ausgestattet. Diese Einrichtung 28 ist als Dichtungsring ausgestaltet, in den Gas (z.B. Luft) bzw. Flüssigkeit (bevorzugt frisches Wasser aus dem externen Reservoir 16, um das Innere des Dichtungsrings sauber zu halten) eingebracht werden kann, um den Dichtungsring auszudehnen und dadurch den Anschluss 17 zu verschließen. Bei Bedarf (z.B. aufgrund von Beschädigungen), kann nun das genannte Paar von Steig- und Fallrohr 18a bzw. 18b ausgetauscht werden, ohne dass der Fluss des Kulturmediums 6 im Rohr 3 gestört wird.

Um nun das Paar von Steig- und Fallrohr 18a bzw. 18b zu reinigen, wird aus einer Kammer in Modul 37b ein silikonbeschichteter, elastischer Ball 29 in Steigrohr 18a gepumpt. Durch Aufrechterhaltung des Pumpendrucks durchläuft der Ball 29 das gesamte Steigrohr 18a, das Verbindungsstück zu Fallrohr 18b und wird von einer baugleichen Kammer in Modul 37a wieder aufgenommen, wobei Anlagerungen von den Innenwänden von Steig- und Fallrohr und Verbindungsstück entfernt werden. Daraufhin wird kann die Instandhaltungsvorrichtung zum nächsten Paar von Steig- und Fallrohr 18a bzw. 18b gezogen werden, wo der beschriebene Abdichtungs- und Reinigungsvorgang wiederholt wird (jedoch mit umgekehrter Pumprichtung, da der Ball 29 sich nun in der Kammer von Modul 37a befindet - alternativ kann der Ball 29 noch im vorigen Paar von Steig- und Fallrohr 18a bzw. 18b in die Kammer von Modul 37b wieder zurückgepumpt werden, bevor die Instandhaltungsvorrichtung zu diesem nächsten Paar gezogen wird).

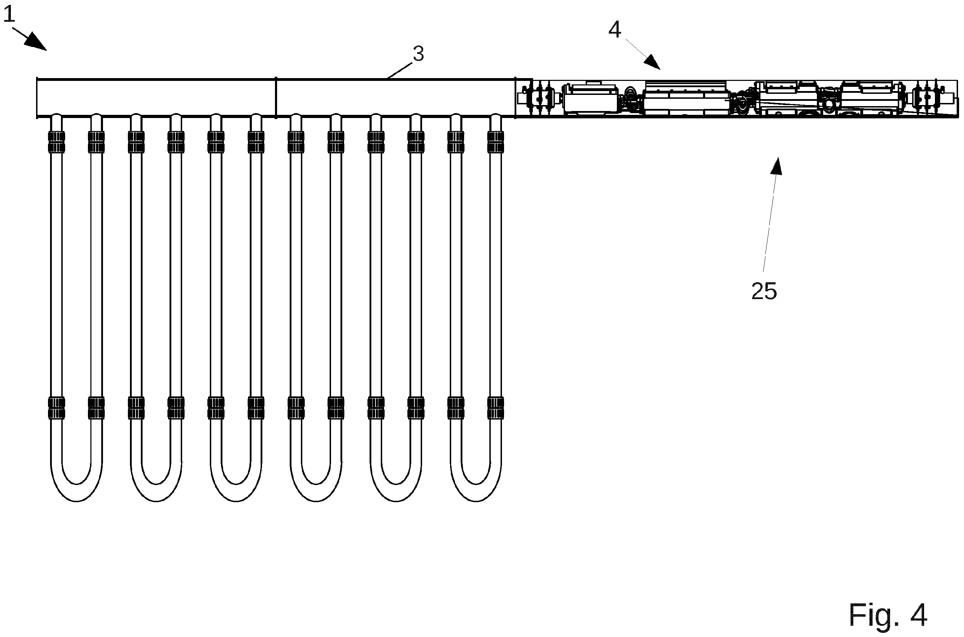

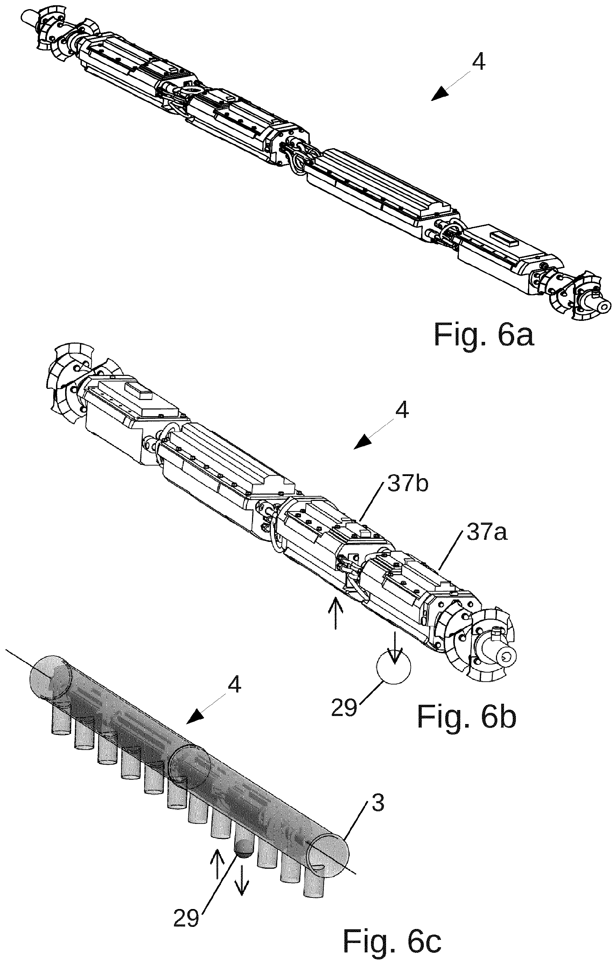

Fig. 4 zeigt schematisch den Photobioreaktor 1 mit der Instandhaltungsvorrichtung 4 in der Station 25, die direkt aus dem Rohr 3 befahrbar ist. Diese Station 25 vereinfacht die Entnahme bzw. das Wiedereinsetzen der Instandhaltungsvorrichtung 4. Die Station 25 kann ein höheres Niveau als das Rohr 3 aufweisen, damit das darin befindliche Kulturmedium nicht in die Station 25 fließt. Wenn vorgesehen ist, dass das Rohr 3 im Betrieb z.B. nur bis zur halben Höhe gefüllt ist, ist z.B. auch denkbar, dass die Station 25 dasselbe Niveau wie das Rohr 3 aufweist (sozusagen eine Verlängerung des Rohres 3 ist). Ferner weist die Station 25 einen Sterildeckel und einen teilbaren Deckel auf, der die Entnahmemöglichkeit der Instandhaltungsvorrichtung 4 und die Verschließbarkeit des Rohres 3 gewährleistet. Fig. 5 zeigt eine bevorzugte Ausführung der erfindungsgemäßen Instandhaltungsvorrichtung 4, mit den Modulen 37a-37d. An den Modulen 37a und 37d sind Elemente 39 mit jeweils drei Reihen von Wischblättern 14 aus Silikonkautschuk angebracht. Wie auch aus der Fig. 6A bzw. 6B ersichtlich, sind diese drei Reihen in Bezug zueinander verdrehbar und decken jeweils nicht den gesamten Rohrquerschnitt ab, so dass die Instandhaltungsvorrichtung 4 umströmbar bleibt. An den Modulen 37a und 37d ist darüber hinaus jeweils eine Sprühdüse 12 zur Reinigung der Innenoberfläche des Photobioreaktor-Rohres vorgesehen. Das Element 39 weist eine Sprühdüse 12a auf, die auf die Wischblätter 14 gerichtet ist. Das distale Ende von Element 39 dient dazu, das Seil des Antriebsystems 5 mit Seilwinde 22 zu verankern. Da die Leitung mit der Reinigungsflüssigkeit im Seil vorgesehen ist, befindet sich auch der Anschluss 26 für eine Leitung mit der Reinigungsflüssigkeit am genannten Ende.

Mit dieser Instandhaltungsvorrichtung 4 kann der gesamte Querschnitt eines Photobioreaktor-Rohres (Fläche in Kontakt mit dem Gasraum über dem Kulturmedium - mit etwaigen abgelagerten Mikroorganismen, Fläche in Kontakt mit der Grenzfläche zwischen Gasraum und Kulturmedium und Fläche in Kontakt mit Kulturmedium - mit etwaigen Biofilmen) simultan gereinigt werden, wenn sie durch das Rohr gezogen wird.

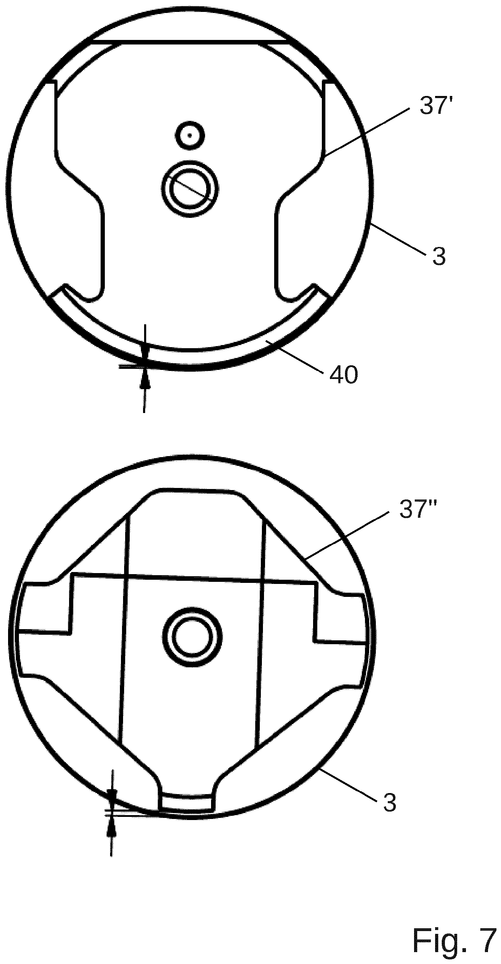

Die Module 37a und 37b sind im Wesentlichen baugleich, jedoch entgegengesetzt orientiert. Sie sind jeweils mit Magnetventilen 33 und einer Einrichtung 28 zum Abdichten von Anschlüssen des Rohres ausgestattet. Mit der Einrichtung 28 verbunden ist jeweils die Kammer 35, in der ein freisetzbares Reinigungsinstrument 29 wie z.B. ein Schwamm aufbewahrt wird, der in an das Rohr angeschlossene, weitere Rohre gepumpt werden kann. Das Modul 37c beinhaltet eine Membranpumpe 32 und weitere Magnetventile 33. Zuletzt beherbergt das Modul 37d die Steuerungselektronik. Auch anhand der Figur 7 wird veranschaulicht, dass alle der genannten Module vom Kulturmedium umströmbar sind.

Fig. 6a zeigt eine perspektivische Ansicht der soeben beschriebenen Ausführung der Instandhaltungsvorrichtung 4. Fig. 6b ebenso, wobei zusätzlich noch veranschaulicht wird, wo das Reinigungsinstrument 29 aus dem Modul 37a freigesetzt wird und vom im Wesentlichen baugleichen Modul 37b wieder aufgenommen wird. Fig. 6c zeigt, wie dieser Vorgang im Rohr 3 vonstatten geht.

Fig. 7 zeigt beispielhaft Querschnitte von Modulen 37' bzw. 37" der Instandhaltungsvorrichtung im Rohr 3. Modul 37' steht mit Rohr 3 über eine Oberfläche 40 aus Silikonkautschuk in Berührungen, was beim Bewegen der Instandhaltungsvorrichtung einen zusätzlichen Reinigungseffekt hat. Durch die gezeigten Querschnitte ist sichergestellt, dass das Kulturmedium mit den Mikroorganismen im Rohr 3 die Module 37' bzw. 37" im Wesentlichen ungestört umströmen kann.

Anhand der folgenden Ausführungsbeispiele wird der Betrieb von besonders bevorzugten Ausführungsformen des erfindungsgemäßen Photobioreaktors weiter veranschaulicht:
Fig. 8 zeigt einen Photobioreaktor 1 zur Kultivierung von phototrophen Mikroorganismen, mit zwei Reaktorelementen 2, die jeweils eine Vielzahl von vertikalen, geraden Steigrohren 18a und Fallrohren 18b für Kulturmedium 6 und ein Rohr 3, welches horizontal, gerade und als Verteilrohr ausgebildet ist, aufweisen. Das Kulturmedium 6 ist ein flüssiges Kulturmedium auf Wasserbasis und angereichert mit Nährstoffen; es beinhaltet die phototrophen Mikroorganismen (üblicherweise Mikroalgen). Das Rohr 3 und die Steig- bzw. Fallrohre 18a bzw. 18b bestehen aus Glas, wobei die Rohre 3 mit als Anschlussfortsätzen ausgebildeten Anschlüssen 17 vorgesehen sind (vgl. auch Fig. 11A und Fig. 11B). Die Steigrohre 18a und Fallrohre 18b sind jeweils an ihrem oberen Ende 19 mit Hilfe der Rohrverbinder 51 mit den als Anschlussfortsätzen ausgebildeten Anschlüssen 17 des als Verteilrohr ausgebildeten Rohres 3 flüssigkeitsdurchlässig verbunden. Die Steigrohre 18a und die Fallrohre 18b sind zusätzlich miteinander durch je ein U-förmiges Verbindungsstück 20 flüssigkeitsdurchlässig verbunden (vgl. auch Fig. 10).

Der Photobioreaktor 1 weist ferner eine Einrichtung 7 zur Begasung des Kulturmediums mit einen kohlendioxidhaltigen Gasgemisch ("Gas") auf, wobei das Gas in die Steigrohre 18a bzw. die Fallrohre 18b eingeleitet wird. Für das mit dem Fallrohr 18b durch das Verbindungsstück 20 verbundene Steigrohr 18a ist ein Einlass 114a zur Begasung durch die Einrichtung 7 vorgesehen. Für das mit dem Steigrohr 18a durch das Verbindungsstück 20 verbundene Fallrohr 18b ist ein weiterer Einlass 114b zur Begasung durch die Einrichtung 7 vorgesehen. Das Kohlendioxid wird aus einem Flüssigtank in ein Gasverteilrohr eingeleitet. Die Anschlüsse des Gasverteilrohres sind durch Schläuche mit je einem Einlass 114a bzw. 114b verbunden. Durch Vorsehen einer Öffnung des Einlasses 114a, die durchlässiger ist als die Öffnung des Einlasses 114b, oder durch unterschiedliche Gasdrücke in 2 voneinander getrennten Gasverteilrohren wird eine stärkere Begasung der Steigrohre 18a gegenüber den Fallrohren 18b ermöglicht. Dadurch kommt es -obwohl am jeweiligen oberen Ende 20 der Steigrohre 18a bzw. Fallrohre 18b keine U-förmigen Verbindungsstücke 20 sondern das als Verteilrohr ausgebildete Rohr 3 vorgesehen ist - zu einer mäanderförmigen Strömung des Kulturmediums 6 (aufwärts in den Steigrohren 18a, abwärts in den Fallrohren 18b; vgl. auch die gestrichelten Pfeile in Fig. 9). Diese mäanderförmige Strömung wird darüber hinaus weiter stabilisiert, indem die zwei Reaktorelemente 2 über ein Fallrohr 18b des ersten Reaktorelements 2 mit einem Steigrohr 18b des zweiten Reaktorelements 2 miteinander durch ein U-förmiges Verbindungsstück 20 flüssigkeitsdurchlässig verbunden sind - also vereinfacht gesagt durch Vorsehen einer "Lücke" zwischen den beiden als Verteilrohren ausgebildeten Rohren 3.

Der Einlass 52a und der Auslass 52b können miteinander verbunden werden, wodurch ein zyklischer Betrieb des Photobioreaktors 1 ermöglicht wird. Nach einer gewissen Anzahl an Zyklen unter Belichtung kann das Kulturmedium 6, das nun eine deutlich höhere Konzentration an Mikroorganismen enthält, am Auslass 52b zur Ernte (d.h. zur Aufkonzentration und Trocknung der phototrophen Mikroorganismen) entnommen werden, während am Einlass 52a frisches Kulturmedium 6 mit einer niedrigen Anfangskonzentration an phototrophen Mikroorganismen eingebracht wird. Selbstverständlich ist auch ein kontinuierlicher zyklischer Betrieb denkbar, bei dem der Einlass 52a mit dem Auslass 52b durch einen Schlauch verbunden bleibt, und an einem Einlass in einem ersten unteren Verbindungsstück 20 kontinuierlich frisches Kulturmedium 6 zugeführt wird und an einem Auslass in einem ausgehend von der Strömungsrichtung direkt davor liegenden zweiten unteren Verbindungsstück 20 kontinuierlich dieselbe Menge dichteres Kulturmedium 6 entnommen wird. Dafür ist naturgemäß eine gewisse Mindestlänge der Zuchtstrecke erforderlich. Ebenso ist, wenn der Photobioreaktor 1 eine gewisse Mindestlänge aufweist, auch ein kontinuierlicher linearer Betrieb denkbar, bei dem kontinuierlich frisches Kulturmedium 6 mit einer niedrigen Anfangskonzentration an phototrophen Mikroorganismen beim Einlass 52a eingebracht wird und kontinuierlich dieselbe Menge reifes Kulturmedium 6 am Auslass 52b zur Ernte entnommen wird. Einlässe können jedoch auch an anderen Stellen des Reaktors vorgesehen werden (z.B. U-Bogen-Verbindungsstück 20 des ersten/letzten Steig- bzw. Fallrohres 18a bzw. 18b, aber auch in einem anderen U-Bogen-Verbindungsstück 20 im Photobioreaktor).

Das mäanderförmig strömende Kulturmedium 6 mit den phototrophen Mikroorganismen steht in den Reaktorelementen 2 so hoch, dass auch die als Verteilrohre ausgebildeten Rohre 3 jeweils etwa zur Hälfte damit befüllt sind. Im Rohr 3 sind also sowohl Kulturmedium 6 als auch, oberhalb des Kulturmediums 6, ein Gasraum 9 zur Aufnahme von aus dem Kulturmedium 6 aufsteigenden Gasblasen 8 vorhanden, wobei im Rohr 3 eine Grenzfläche 10 zwischen dem Kulturmedium 6 und dem Gasraum 9 angeordnet ist. Der Gasdruckausgleich mit der Umgebung findet über eine Öffnung 53 statt, welche mit einem Filtersystem ausgestattet ist, um Kontamination des Kulturmediums 6 zu vermeiden.

Die mit der Grenzfläche 10 zwischen Kulturmedium 6 und Gasraum 9 in Zusammenhang stehenden Verschmutzungen an der Innenoberfläche des Rohres 3 könnten mit im Vergleich zum Stand der Technik (der die Grenzfläche nicht in einem Verteilrohr vorsieht, sondern an viel schwieriger zu erreichenden Stellen; vgl. z.B. die US 2011/0027875 A1, insbesondere den unteren Teil von Fig. 1 darin) verhältnismäßig geringem Aufwand gereinigt werden, indem der Photobioreaktor 1 kurz außer Betrieb genommen wird, das Kulturmedium 6 aus dem Rohr 3 abgelassen wird, die Abdeckplatte 54 des Rohres 3 entfernt wird und die Innenoberfläche das Rohres 3 z.B. mit einer an einer Teleskopstange befestigten Bürste gereinigt wird. Zu viel besseren Resultaten führt jedoch der Einsatz der erfindungsgemäß vorgesehenen Instandhaltungsvorrichtung 4, wie auch im Folgenden beschrieben.

Die in Fig. 9 gezeigte Instandhaltungsvorrichtung 4 im Photobioreaktors 1 ermöglicht die Reinigung bzw. das Sauberhalten der inneren Oberfläche des als Verteilrohr ausgebildeten Rohres 3 während des Betriebes. Dabei ist am Rohr 3 die Führungsleiste 21 für die Instandhaltungsvorrichtung 4 vorgesehen. Die Instandhaltungsvorrichtung 13 ist mit einer Sprühdüse 12 zum Besprühen der inneren Oberfläche des Rohres 3 ausgestattet. Die Instandhaltungsvorrichtung 4 weist ein magnetisches Element auf und kann durch die Führungsleiste 21 verdrehsicher positioniert werden. Die Instandhaltungsvorrichtung 4 ist umströmbar (sodass der mäanderförmige Fluss des Kulturmediums 6 nicht wesentlich gestört wird) und kann während des laufenden Betriebes in voreinstellbaren Zeitabständen eingesetzt werden, was die Stehzeiten des Photobioreaktors 1 wesentlich verringert. Auch wenn sie gerade nicht eingesetzt wird, kann die Instandhaltungsvorrichtung 4 im Rohr 3 verbleiben. Die gestrichelten Pfeile verdeutlichen den Strömungspfad im Reaktorelement 2.

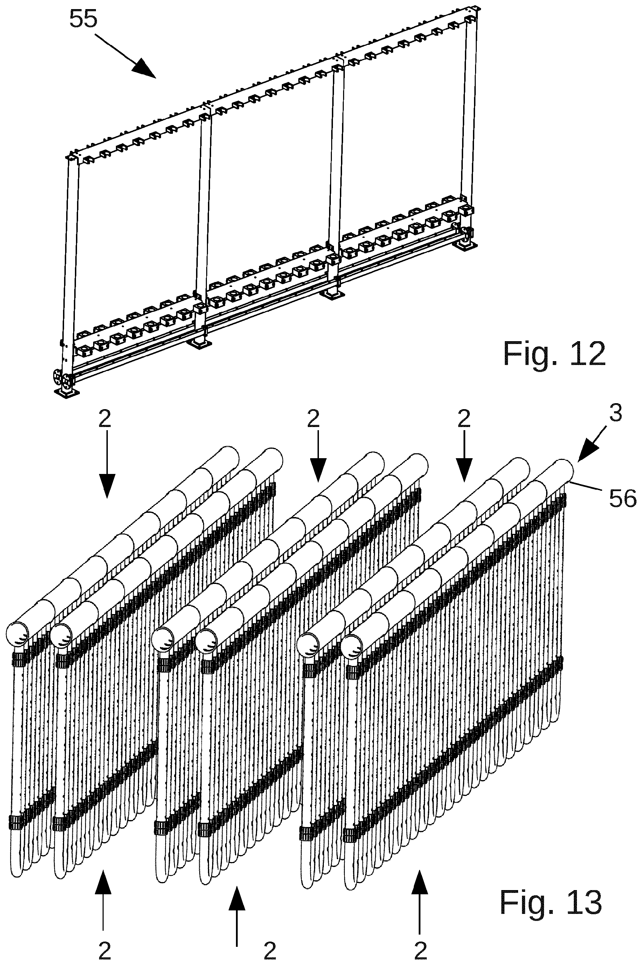

Fig. 13 zeigt schematisch eine Ausführungsform des Photobioreaktors 1, bei der das jeweilige als Verteilrohr ausgebildete Rohr 3 eines Reaktorelements 2 aus miteinander direkt verbundenen Glasteilstücken 56 besteht. Jeweils zwei Reaktorelemente 2 werden durch das in Fig. 12 gezeigte Metallgerüst 55 gehalten.

## Patentansprüche

1. Photobioreaktor (1) zur Kultivierung phototropher Mikroorganismen,
mit einem Reaktorelement (2), das ein Rohr (3) aufweist, und mit einer Instandhaltungsvorrichtung (4) und einem Antriebssystem (5), das die Instandhaltungsvorrichtung (4) im Rohr (3) bewegen kann,
wobei der Photobioreaktor (1) derart ausgelegt ist, dass das Rohr (3) im Betriebszustand des Photobioreaktors (1) von flüssigem Kulturmedium (6) mit den Mikroorganismen zumindest teilweise durchströmt wird,
wobei der Photobioreaktor (1) derart ausgelegt ist, dass die Instandhaltungsvorrichtung (4) im Betriebszustand des Photobioreaktors (1) im Rohr (3) einsetzbar ist und vom Antriebssystem (5) zumindest gegen die Strömung des Kulturmediums (6) im Rohr (3) bewegt werden kann,
**dadurch gekennzeichnet, dass** das Rohr (3) ein Verteilrohr mit zumindest drei Anschlüssen (17) für den Zufluss bzw. Abfluss von Kulturmedium (6) ist, und der Photobioreaktor (1) derart ausgelegt ist, dass die Instandhaltungsvorrichtung (4) zu zumindest einem der Anschlüsse im Rohr (3) bewegt werden kann und diesen abdichten kann,
wobei das Reaktorelement (2) ferner eine Vielzahl von Steig-(18a) und Fallrohren (18b) für das flüssiges Kulturmedium (6) aufweist, wobei die Steig- (18a) und Fallrohre (18b) jeweils an ihrem oberen Ende (19) an das als Verteilrohr ausgebildete Rohr (3) flüssigkeitsdurchlässig angeschlossen sind, wobei zumindest eines der Steig- (18a) und eines der Fallrohre (18b) zusätzlich miteinander durch ein Verbindungsstück (20) flüssigkeitsdurchlässig verbunden sind, und
wobei der Photobioreaktor (1) derart ausgelegt ist, dass die Instandhaltungsvorrichtung (4) gleichzeitig den Anschluss (17) für das Fallrohr (18b) und den Anschluss (17) für das Steigrohr (18a), welche beide miteinander zusätzlich durch ein Verbindungsstück (20) flüssigkeitsdurchlässig verbunden sind, abdichten kann.

2. Photobioreaktor (1) gemäß Anspruch 1, **dadurch gekennzeichnet, dass** der Photobioreaktor (1) ferner eine Einrichtung (7) zur Begasung des Kulturmediums aufweist, wobei der Photobioreaktor (1) derart ausgelegt ist, dass im Betriebszustand des Photobioreaktors (1) die Instandhaltungsvorrichtung (4) im Rohr (3) mit im Kulturmedium (6) befindlichen Gasblasen (8) in Berührung gebracht wird.

3. Photobioreaktor (1) gemäß einem der Ansprüche 1 bis 2, **dadurch gekennzeichnet, dass** der Photobioreaktor (1) derart ausgelegt ist, dass im Betriebszustand im Rohr (3) sowohl Kulturmedium (6) als auch, oberhalb des Kulturmediums, ein Gasraum (9) zur Aufnahme von aus dem Kulturmedium (6) aufsteigenden Gasblasen (8) vorhanden sind, wobei im Rohr (3) eine Grenzfläche (10) zwischen dem Kulturmedium (6) und dem Gasraum (9) angeordnet ist, und die Instandhaltungsvorrichtung (4) zumindest zur Reinigung von mit dem Gasraum (9) in Berührung befindlicher innerer Oberfläche (11a) des Rohres (3) eingerichtet ist.

4. Photobioreaktor (1) gemäß einem der Ansprüche 1 bis 3, **dadurch gekennzeichnet, dass** die Instandhaltungsvorrichtung (4) eine Sprühdüse (12) zum Besprühen der inneren Oberfläche (11) des Rohres (3) mit einer Reinigungsflüssigkeit (13) aufweist, vorzugsweise wobei die Instandhaltungsvorrichtung (4) zumindest zum Besprühen von mit dem genannten Gasraum (9) in Berührung befindlicher innerer Oberfläche (11a) des Rohres (3) durch die Sprühdüse (12) eingerichtet ist.

5. Photobioreaktor (1) gemäß Anspruch 4, wobei die Sprühdüse (12) über eine Leitung (15) mit einem Flüssigkeitsreservoir (16) außerhalb des Rohres (3) verbunden ist.

6. Photobioreaktor (1) gemäß einem der Ansprüche 1 bis 5, **dadurch gekennzeichnet, dass** die Instandhaltungsvorrichtung (4) dazu eingerichtet ist, das durch die Instandhaltungsvorrichtung (4) gegenüber dem Rohr (3) abgedichtete Fallrohr (18b) und das durch die Instandhaltungsvorrichtung (4) gegenüber dem Rohr (3) abgedichtete Steigrohr (18a), welche beide miteinander durch ein Verbindungsstück (20) flüssigkeitsdurchlässig verbunden sind, zu reinigen.

7. Photobioreaktor (1) gemäß einem der Ansprüche 1 bis 6, **dadurch gekennzeichnet, dass** das Antriebssystem (5) zumindest eine Seilwinde (22), die ein im Rohr (3) geführtes und mit der Instandhaltungsvorrichtung (4) verbundenes Seil (23) antreiben kann, aufweist,
vorzugsweise wobei die Seilwinde (22) mit einer Einrichtung zur Desinfektion (24) des Seiles (22) ausgestattet ist.

8. Verfahren zur Kultivierung phototropher Mikroorganismen in einem Photobioreaktor,
wobei der Photobioreaktor (1) mit einem Reaktorelement, das ein Rohr (3) aufweist, und mit einer Instandhaltungsvorrichtung (4) im Rohr (3) und einem Antriebssystem, das die Instandhaltungsvorrichtung (4) im Rohr (3) bewegt, ausgestattet ist,
wobei das Rohr (3) von flüssigem Kulturmedium (6) mit den Mikroorganismen zumindest teilweise durchströmt wird,
wobei das Verfahren den Einsatz der Instandhaltungsvorrichtung (4) im Rohr (3) im Betriebszustand des Photobioreaktors (1) umfasst und die Instandhaltungsvorrichtung (4) vom Antriebssystem (5) zumindest gegen die Strömung des Kulturmediums (6) im Rohr (3) bewegt wird,
wobei der Photobioreaktor (1) ferner gemäß einem der Ansprüche 1 bis 7 definiert ist.

## Claims

1. A photobioreactor (1) for culturing phototrophic microorganisms,
with a reactor element (2), which has a tube (3), and with a maintenance device (4) and a drive system (5) which can move the maintenance device (4) in the tube (3),
wherein the photobioreactor (1) is adapted such that, in the operating state of the photobioreactor (1), the tube (3) is at least partially passed through by liquid culture medium (6) with the microorganisms,
wherein the photobioreactor (1) is adapted such that, in the operating state of the photobioreactor (1), the maintenance device (4) is insertable into the tube (3) and can be moved by the drive system (5) at least against the flow of the culture medium (6) in the tube (3),
**characterized in that** the tube (3) is a distributor tube with at least three ports (17) for the inflow or outflow of culture medium (6), and **in that** the photobioreactor (1) is adapted such that the maintenance device (4) can be moved to at least one of the ports in the tube (3) and can seal the same,
wherein the reactor element (2) further has a plurality of risers (18a) and downcomers (18b) for the liquid culture medium (6), wherein the risers (18a) and downcomers (18b) are each connected to the tube (3) formed as a distributor tube at their upper ends (19) in a liquid-permeable manner, wherein at least one of the risers (18a) and one of the downcomers (18b) are additionally connected to each other by a connecting piece (20) in a liquid-permeable manner, and
wherein the photobioreactor (1) is adapted such that the maintenance device (4) can seal the port (17) for the downcomer (18b) and the port (17) for the riser (18a), which are both additionally connected to each other by a connecting piece (20) in a liquid-permeable manner, at the same time.

2. The photobioreactor (1) according to claim 1, **characterized in that** the photobioreactor (1) further has means (7) for gassing the culture medium, wherein the photobioreactor (1) is adapted such that, in the operating state of the photobioreactor (1), the maintenance device (4) in the tube (3) is contacted with gas bubbles (8) present in the culture medium (6).

3. The photobioreactor (1) according to any one of claims 1 to 2, **characterized in that** the photobioreactor (1) is adapted such that, in the operating state, both culture medium (6) and, above the culture medium, a gas space (9) for receiving gas bubbles (8) rising from the culture medium (6) are present in the tube (3), wherein an interface (10) between the culture medium (6) and the gas space (9) is arranged in the tube (3), and the maintenance device (4) is at least configured for cleaning an inner surface (11a) of the tube (3) in contact with the gas space (9).

4. The photobioreactor (1) according to any one of claims 1 to 3, **characterized in that** the maintenance device (4) has a spray nozzle (12) for spraying the inner surface (11) of the tube (3) with a cleaning liquid (13),
preferably wherein the maintenance device (4) is at least arranged for spraying an inner surface (11a) of the tube (3) in contact with said gas space (9) through the spray nozzle (12).

5. The photobioreactor (1) according to claim 4, wherein the spray nozzle (12) is connected to a liquid reservoir (16) outside of the tube (3) via a line (15).

6. The photobioreactor (1) according to any one of claims 1 to 5, **characterized in that** the maintenance device (4) is configured to clean the downcomer (18b) sealed by the maintenance device (4) relative to the tube (3) and the riser (18a) sealed by the maintenance device (4) relative to the tube (3), which are both connected to each other by a connecting piece (20) in a liquid-permeable manner.

7. The photobioreactor (1) according to any one of claims 1 to 6, **characterized in that** the drive system (5) has at least one cable winch (22), which can drive a cable (23) guided in the tube (3) and connected to the maintenance device (4),
preferably wherein the cable winch (22) is equipped with means for disinfecting (24) the cable (22).

8. A method of culturing phototrophic microorganisms in a photobioreactor,
wherein the photobioreactor (1) is equipped with a reactor element, having a tube (3), and with a maintenance device (4) in the tube (3) and a drive system which moves the maintenance device (4) in the tube (3),
wherein the tube (3) is at least partially passed through by liquid culture medium (6) with the microorganisms,
wherein the method comprises the use of the maintenance device (4) in the tube (3) in the operating state of the photobioreactor (1), and the maintenance device (4) is moved by the drive system (5) at least against the flow of the culture medium (6) in the tube (3),
wherein the photobioreactor (1) is further defined according to any one of claims 1 to 7.

## Revendications

1. Photobioréacteur (1) pour la culture de microorganismes phototrophes, avec un élément réacteur (2) qui présente un tuyau (3), et avec un dispositif de maintenance (4) et un système d'entraînement (5) pouvant déplacer le dispositif de maintenance (4) dans le tuyau (3),
le photobioréacteur (1) étant conçu de manière à ce que le tuyau (3) en l'état d'exploitation du photobioréacteur (1) soit traversé au moins en partie par un milieu de culture fluide (6) avec les microorganismes,
le photobioréacteur (1) étant conçu de manière à ce que le dispositif de maintenance (4) en l'état d'exploitation du photobioréacteur (1) soit utilisable dans le tuyau (3) et puisse être déplacé par le système d'entraînement (5) au moins dans le sens opposé à l'écoulement du milieu de culture (6) dans le tuyau (3),
**caractérisé en ce que** le tuyau (3) est un tuyau de distribution avec au moins trois raccords (17) pour l'arrivée et/ou la sortie du milieu de culture (6), et le photobioréacteur (1) est conçu de manière à ce que le dispositif de maintenance (4) puisse être déplacé vers au moins l'un des raccords dans le tuyau (3) et puisse le rendre étanche,
l'élément réacteur (2) présentant en outre une pluralité de tuyaux montants (18s) et descendants (18b) pour le milieu fluide de culture (6), les tuyaux montants (18a) et descendants (18b) étant chacun raccordés de manière perméable aux fluides par leur extrémité supérieure (19) au tuyau (3) formant le tuyau de distribution, au moins l'un des tuyaux montants (18a) et l'un des tuyaux descendants (18b) étant en plus reliés l'un à l'autre de manière perméable aux fluides par un élément de liaison (20), et
le photobioréacteur (1) étant conçu de manière à ce que le dispositif de maintenance (4) puisse rendre étanche simultanément le raccord (17) pour le tuyau descendant (18b) et le raccord (17) pour le tuyau montant (18a), les deux étant en plus reliés l'un à l'autre de manière perméable aux fluides par un élément de liaison (20).

2. Photobioréacteur (1) selon la revendication 1, **caractérisé en ce que** le photobioréacteur (1) présente en outre un dispositif (7) pour le gazage du milieu de culture, le photobioréacteur (1) étant conçu de manière à ce qu'en l'état d'exploitation du photobioréacteur (1), le dispositif de maintenance (4) dans le tuyau (3) soit mis en contact avec des bulles de gaz (8) se trouvant dans le milieu de culture (6).

3. Photobioréacteur (1) selon l'une quelconque des revendications 1 à 2, **caractérisé en ce que** le photobioréacteur (1) est conçu de manière à ce qu'en l'état d'exploitation dans le tuyau (3), le milieu de culture (6) ainsi que, au-dessus du milieu de culture, un espace gazeux (9) pour recevoir les bulles de gaz (8) qui montent du milieu de culture (6) soient présents, une surface de délimitation (10) étant disposée dans le tuyau (3) entre le milieu de culture (6) et l'espace gazeux (9), et le dispositif de maintenance (4) est agencé au moins pour nettoyer la surface interne (11a) du tuyau (3) qui est en contact avec l'espace gazeux (9).

4. Photobioréacteur (1) selon l'une quelconque des revendications 1 à 3, **caractérisé en ce que** le dispositif de maintenance (4) présente une buse de pulvérisation (12) pour pulvériser la surface interne (11) du tuyau (3) avec un fluide de nettoyage (13), de préférence le dispositif de maintenance (4) étant agencé au moins pour pulvériser par la buse de pulvérisation (12) la surface interne (11a) du tuyau (3) en contact avec ledit espace gazeux (9).

5. Photobioréacteur (1) selon la revendication 4, la buse de pulvérisation (12) étant reliée par une conduite (15) à un réservoir de fluide (16) à l'extérieur du tuyau (3).

6. Photobioréacteur (1) selon l'une quelconque des revendications 1 à 5, **caractérisé en ce que** le dispositif de maintenance (4) est agencé pour nettoyer le tuyau descendant (18b) rendu étanche au tuyau (3) par le dispositif de maintenance (4) et le tuyau montant (18a) rendu étanche au tuyau (3) par le dispositif de maintenance (4) et qui sont reliés l'un à l'autre de manière perméable aux fluides par un élément de liaison (20).

7. Photobioréacteur (1) selon l'une quelconque des revendications 1 à 6, **caractérisé en ce que** le système d'entraînement (5) présente au moins un treuil à câble (22) qui peut entraîner un câble (23) guidé dans le tuyau (3) et relié au dispositif de maintenance (4),
de préférence le treuil à câble (22) étant équipé d'un dispositif pour la désinfection (24) du câble (22).

8. Procédé de culture de microorganismes phototrophes dans un photobioréacteur,
le photobioréacteur (1) étant équipé d'un élément réacteur qui présente un tuyau (3) et d'un dispositif de maintenance (4) dans le tuyau (3) et d'un système d'entraînement qui déplace le dispositif de maintenance (4) dans le tuyau (3),
le tuyau (3) étant traversé au moins en partie par le milieu fluide de culture (6) avec les microorganismes,
le procédé comprenant l'utilisation du dispositif de maintenance (4) dans le tuyau (3) en l'état d'exploitation du photobioréacteur (1) et le dispositif de maintenance (4) étant déplacé par le dispositif d'entraînement (5) au moins dans le sens opposé à l'écoulement du milieu de culture (6) dans le tuyau (3),
le photobioréacteur (1) étant en outre défini selon l'une quelconque des revendications 1 à 7.
